# EUROPEAN PATENT SPECIFICATION

(11) **EP 1 534 185 B1**
(45) Date of publication and mention of the grant of the patent: **16.09.2015**
(21) Application number: 03788613.2
(22) Date of filing: 15.08.2003
(51) Int. Cl.: A61F 2/06

(54) **STENT AND METHOD OF FORMING A STENT WITH INTEGRAL BARBS**
STENT UND VERFAHREN ZUR BILDUNG EINES STENTS MIT INTEGRALEN WIDERHAKEN
STENT ET PROCEDE PERMETTANT DE FORMER UN STENT A BARBES MONOBLOC

(30) Priority: 15.08.2002 US 403783 P
(43) Date of publication of application: 01.06.2005
(73) Proprietor: Cook Medical Technologies LLC, Bloomington, IN 47404 (US)
(72) Inventor: MEAD, JASON A, Indianapolis IN 46217 (US); OSBORNE, THOMAS, A, Bloomington IN 47401 (US)
(74) Representative: Garratt, Peter Douglas
(86) International application number: PCT/US2003/025827
(87) International publication number: WO 2004/016201

(56) References cited:
- EP-A- 0 539 237
- WO-A-01/56500
- WO-A-01/76509
- US-A- 5 843 164
- US-A1- 2002 055 772

## Description

### TECHNICAL FIELD

The present invention relates to medical devices and, in particular, a method of forming a stent for implantation within human or animal bodies for the repair of damaged vessels such as blood vessels.

The present invention also relates to the design and manufacture of stents for implantation into blood vessels or other lumens. These stents have barbs that are integral with the stent wire; in other words, they have not been attached to the stent wire during the manufacturing process. This invention also relates to medical devices, more particularly to stents and other prosthetic and/or intraluminal devices having anchoring barbs.

### BACKGROUND OF THE INVENTION

The functional vessels of human and animal bodies such as blood vessels and ducts can occasionally weaken. For example, the aortic wall can weaken, resulting in an aneurysm. Upon further exposure to haemodynamic forces, such an aneurysm can rupture.

These medical conditions and similar pathologies can call for surgical intervention. The aneurismal aorta, for example, may be treated using an endoluminal prosthesis. Such an endoluminal prosthesis will exclude the aneurysm so that the aneurysm no longer grows, nor has the opportunity to rupture.

One endoluminal prosthesis which is useful for repair of aortic aneurysms is disclosed in PCT application WO98/53761. This application discloses a prosthesis which includes a sleeve or tube of biocompatible prosthesis material such as DACRON^{®} polyester fabric (trademark of E. I. DuPont de Nemours and Co.) or polytetrafluoroethylene (PTFE) defining a lumen. The WO98/53761 prosthesis further includes several zigzag stents secured therealong. These stents can be, for example, Gianturco Z-stents, which are commercially available from Cook Inc., Bloomington, Indiana.

The prosthesis of the PCT application WO98/53761 is designed to span an aneurysm that extends along the aorta proximally from the two iliac arteries. This application also discloses the manner of deploying the stent prosthesis in the patient utilizing an introducer assembly.

In the WO98/53761 application, the material-covered portion of the single-lumen proximal end of the prosthesis bears against the wall of the aorta above the aneurysm to seal the aneurysm at a location that is spaced distally of the entrances to the renal arteries. Thin wire struts of a proximal fixation stent traverse the renal artery entrances without occluding them, since no prosthesis material is utilized along the proximal stent. The fixation stent secures the stent prosthesis in position within the aorta when the stent self-expands.

Blood vessels and other vessels can also become stenotic or occluded. For example, arteries can develop atherosclerotic plaques which can cause stenosis; eventually, a stenotic artery can become completely occluded. A stenotic or occluded artery can be treated by introducing self-expanding, balloon- expandable or shape-memory stents which expand the lumen at the site of stenosis or occlusion. Such a stent is disclosed in U.S., Patent No. 6,464,720.

U.S. Patent No. 6,464,720 discloses an expandable antistenotic stent made from a cannula or sheet of biocompatible material that includes at least one longitudinal segment comprised of a series of laterally interconnected closed cells. Each closed cell of a longitudinal segment is defined laterally by a pair of longitudinal struts that are interconnected at each end by a circumferentially adjustable member. When the stent is expanded using a balloon, the opposing circumferentially adjustable members deform to allow circumferential expansion of the longitudinal segment, while the length of the segment, as defined by the longitudinal struts, is maintained. Self-expanding versions of the stent utilize a nickel-titanium alloy. Other stents are disclosed in U.S. Patent Nos. 5,632,771 and 6,409,752.

When endoluminal prostheses or antistenotic stents are implanted to treat these or similar conditions, it is important that they do not migrate under physiological forces. Pulsatile flow is a major force that stents encounter; thus stents and endoluminal prostheses tend to move downstream in the blood vessel in which they are placed.

If the stents or endoluminal prostheses do migrate, they can travel beyond the length of the vessel they are intended to treat. For example, if an antistenotic stent migrates, it will fail to keep the targeted portion of the vessel from restenosing. If an endoluminal prosthesis migrates, it can expose the aneurysm it was meant to treat. The aneurysm will then repressurize, presenting a risk of rupture.

Migration can be a significant problem in the placement of expandable stents and other intraluminal devices, especially when placed in the arterial region of the vascular system. Nowhere is the prevention of migration more important and more challenging than when placing a stent graft to repair an abdominal aortic aneurysm (AAA) where downstream migration of the device can result in the aneurysm no longer being excluded. If the aneurysm is no longer intact or subsequent rupture were to occur, the patient would then face an increased risk of death. Unlike surgically placed grafts which are sutured into place, only the radial forces of the stent would be available to hold the prosthesis into place.

If an endoluminal prosthesis migrates towards a branch vessel, it can partially or totally occlude it. Likewise, if a fenestrated endoluminal prosthesis migrates, it can occlude the branch vessel to which the fenestration was to permit blood flow. If this happens to a fenestrated thoracic endoluminal prosthesis, for example, important branch vessels (*e.g*. the common carotid) can be occluded, resulting in death. If this happens to an aortic abdominal endoluminal prosthesis with renal artery fenestrations, kidney function can be seriously impaired.

To address the problem of migration, stent graft manufacturers sometimes place a series of barbs or hooks that extend outward from the main body of the prosthesis, typically at its proximal end, either by attaching them to the stent frame with solder or by some other bonding technique, or to the graft material, typically by suturing. These barbs can be attached to the stent wire by wrapping, chemical bonding, welding, brazing, soldering or other techniques. For example, one embodiment of the prosthesis of the PCT application WO98/53761 utilizes barbs which extend from the suprarenal fixation stents to engage the aorta wall, to thereby keep the graft from migrating.

However, barbs attached by these methods have been known to break off or bend because repeated physiological stresses, the cyclical loading caused by cardiovascular pulsatile forces in particular, cause mechanical fatigue and failure of the barb-stent junction. It has been observed that sutures attaching barbed stents to the graft material are subject to breakage due in part to the flexibility of the graft material and the considerable pulsatile forces of arterial blood acting on the device. These forces have been known to directly contribute to the detachment between the graft portion and anchoring stent. If the barbs were bent in the manufacturing process, the barbs are further weakened. Furthermore, the barbs are exposed to a physiological environment which is saline, oxygen-rich and acidic, and therefore tends to weaken the barb and its connection through corrosion.

It has also been further observed that barbs soldered or otherwise attached to the stent frame are subject to fracture, detachment, or other failure, especially when the forces become concentrated at a particular location along the stent graft. Unfortunately, simply making the barbs stronger to prevent fracture can result in increased damage to the anchoring tissue. Furthermore, adding rigidity to any outward-projecting barbs may compromise the ability of the device to be compressed and loaded into a delivery system. The use of multiple barbs can prevent catastrophic migration of the device, especially if there are a very limited number of barb failures. Yet, while a single barb failure should not result in the migration of the device and may not represent a problem clinically, barb fracture or failure is nevertheless currently classified as an adverse event that manufacturers seek to avoid.

One solution to address barb failure was disclosed in U.S. Patent No. 5,720,776 to Chuter et al., depicted in FIG. 1, where the barb includes both a mechanical attachment, as well as the traditional solder bond. The mechanical attachment comprises a helical winding of the basal portion of the barb around a strut of the stent prior to addition of the solder joint to help protect the solder joint from failure. In addition, the barb is made laterally flexible to help accommodate forces acting at the anchor point. These improvements help ensure that the barb does not readily detach from the stent due to a failure of the solder joint alone. While the combination of both solder and a mechanical means to affix the barb to the stent has proved effective in most respects; however, this area of the barb remains most subject to stresses, such as from cyclic load resulting from the pulsatile action of the implant vessel. What is needed is a barb design that is better able to accommodate or distribute bending and shear stresses in order to further reduce the likelihood of barb failure due to fracture.

### SUMMARY OF THE INVENTION

In one aspect of the invention there is a method for manufacturing a barbed stent which comprises providing a sheet of stent material, cutting the sheet to form a stent wire with integral barbs extending therefrom and forming the stent wire into a final stent shape having a longitudinal axis.

In another aspect of the invention, there is a method for manufacturing a barbed stent which comprises providing a sheet of stainless steel, cutting the sheet with a laser to form a stent wire with integral barbs extending therefrom, electro-polishing the stent wire, bending the stent wire to form a zigzag shape and soldering a first end of the stent wire to a second end of the stent wire.

In yet another aspect of the invention, there is a method for manufacturing a barbed stent which comprises providing a cannula of stent material, cutting the cannula to form a stent wire with integral barbs extending therefrom and forming the stent wire into a final stent shape having a longitudinal axis.

In yet another aspect of the invention, there is a method for manufacturing a barbed stent which comprises providing a stainless steel cannula, cutting the cannula with a laser to form a stent wire with integral barbs extending therefrom, electro-polishing the stent wire and bending the stent wire to form a zigzag shape.

In yet another aspect of the invention, there is a barbed stent for deployment within the body of a patient, comprising a wire having at least one bend, wherein each of the at least one bend connects to at least two struts and barbs extending in a generally transverse direction from a longitudinal axis of the stent, wherein the barbs are integral with the wire and configured to engage tissue adjacent the stent.

In yet another aspect of the invention, there is an endoluminal prosthesis, comprising a substantially cannular body having proximal and distal ends and a stent affixed to the substantially cannular body near the proximal end wherein the stent has integral barbs extending therefrom, and wherein the stent and the integral barbs are configured so that they can engage tissue adjacent to the stent when the endoluminal prosthesis is deployed.

In yet another example, there is an illustrative implantable valve that is deployed within a bodily passage, such as a blood vessel or the heart, to regulate or augment the normal flow of blood or other bodily fluids. The valve includes a covering having oppositely facing curvilinear-shaped surfaces (upper and lower) against which fluid traveling in a first or second direction within the bodily passage exerts force to at least partially open or close the valve. At least one outer edge of the covering resiliently engages and exerts force against the wall of the vessel and has an arcuate shape that provides at least a partial seal against the wall.

In yet another example, the covering comprises a plurality of leaflets, each leaflet having a body extending from a wall-engaging outer edge to a free edge which is cooperable with one or more opposing leaflets to prevent flow in one direction, such as retrograde flow, while at least a portion of the leaflets having sufficient flexibility, when in situ to move apart, thereby creating a valve orifice that permits flow in the opposite direction, such as normal blood flow. The outer edge of each leaflet is adapted to engage and resiliently exert force against a wall of the bodily passage such that it extends in both a longitudinal and circumferential directions along the vessel wall to at least partially seal a portion of the vessel lumen, while the free edge of each leaflet traverses the passageway across the diameter of the vessel.

In yet another example, valve includes a frame that is covered by a piece of biocompatible material, preferably an Extracellular Collagen Matrix (ECM) such as small intestinal submucosa (SIS) or another type of submucosa-derived tissue. Other potential biomaterials include allographs such as harvested native valve tissue. The material is slit or otherwise provided with an opening along one axis to form two triangular valve leaflets over a four-sided frame. In the deployed configuration, the leaflets are forced open by normal blood flow and subsequently close together in the presence of backflow to help eliminate reflux. Other configurations include a two-leaflet valve having an oval or elliptically shaped frame, and valves having three or more legs and associated, leaflets, which provide a better distribution of the load exerted by the column of fluid acting on the leaflets.

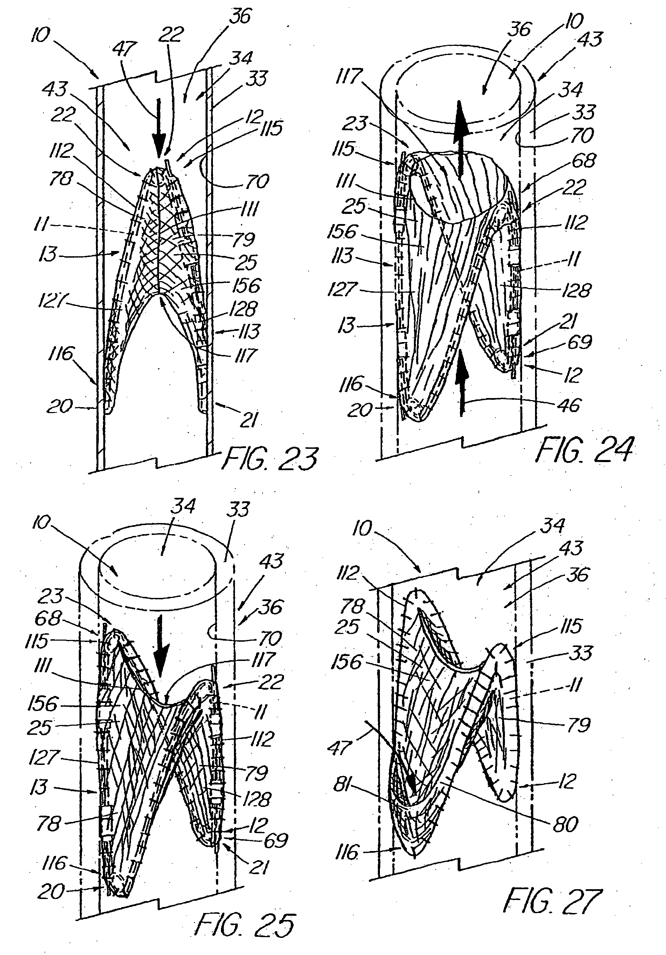

In yet another example, the valve portion of the device, which preferably, but not essentially, includes a saddle-shaped, two-leaflet valve having a serpentine-shaped frame with the resilient outer edges of the leaflets that are sealable about entire circumference of the vessel (as depicted in FIG. 25), further includes additional centering support structure to help align the device within the vessel to prevent tilting that can compromise the performance of the valve. The centering support structure can be separate components attached to the valve portion frame, or be integrally formed with the valve portion frame (e.g., cut from the same piece of cannula).

A first series of examples include centering support structure that extends from the proximal end, distal end, or both ends of the valve portion. This includes, a second (or third) frame, an expandable stent, helical coil, an elongate projection or strut, an inflatable member, extended portion cut from the same cannula used to form the valve portion, or other structure that can be deployed ahead of the valve portion to provide longitudinal support, or remain within the delivery system during deployment of the valve portion, wherein the centering support structure is then also deployed.

A second series of examples include centering support structure, such as a plurality of lateral arms and/or supplemental legs, that extends laterally from the valve portion to provide additional contact points along the circumference of the vessel for longitudinal support. Additionally, the lateral arms, which are preferably positioned behind the leaflets, can offer protection to the leaflets so that they are generally unable to adhere to the vessel wall, which can collapse onto the leaflets due to how the valve radially expands and conforms to the vessel. The lateral arms can comprise separate components attached to the basic valve portion frame, or the frame itself can comprise multiple elements or subassemblies that can be assembled to form a closed valve portion frame with two laterally extending arms. Each lateral arm can include one contact point or additional contact points for added stability.

In another example, the centering support structure comprises two lateral arms, which protect the two leaflets and provide longitudinal support, and two supplemental legs about the distal end of the valve portion for further stabilization to prevent tilting. One method of forming the frame includes attaching two zigzag or serpentine-shaped stents end to end, with struts, sutures, or another well-known mechanism. Each zigzag stent comprises a four or more serpentine sections with at least two opposite sections comprising either lateral arms (proximal stent) or supplemental legs (distal stent), with the other two serpentine sections on each stent comprising a half of one of the valve section legs. Strut lengths, wire diameters, eye diameters, and angles and widths of serpentine sections can be varied to produce optimum radial pressure that the device exerts on the vessel wall, depending on the size of the valve and vessel diameter. The optimal radial pressure is one at which the valve conforms to the vessel and prevents reflux without causing erosion or damage to the vessel wall that could lead to rupture.

In the double serpentine stent example, the covering comprising the leaflets is attached to the frame so that each leaflet spans the two stents with a lateral arm extending outward so that it is external to the leaflet and frame. In an embodiment in which the serpentine stents are attached using a long strut that also adds rigidity to the valve legs which helps prevent partial collapse due to the weight of the blood column, the ends of the struts extend beyond the bends of the valve portion frame to serve as barbs. To help prevent entanglement with the barbs during loading of the device with the delivery system, and modifying radial pressure, the adjacent lateral arms and supplemental legs can be made shorter or longer than the adjacent serpentine sections that comprise the valve legs, so that their respective contact points are offset relative to the ends of the barbs. Additionally, the struts of the serpentine sections can be curved to produce a more rounded configuration for improved conformity with the vessel.

In yet another example, the valve portion is attached inside an expandable stent, or a sleeve of material, such as SIS, that is configured to provide longitudinal stability and prevent tilting. The sleeve can further include an anchoring stent about one end that is deployed ahead of, or after, the valve portion to prevent tilting of the valve.

In yet another example, the frame of the device is modified by placing one or more of the bends under tension which results in the frame assuming a second shape that has superior characteristics of placement within the vessel. One method of adjusting the shape includes forming the bends in the wire at an initial angle, e.g., 150°, that is larger than the desired final angle, e.g., 90° for a four-sided valve, so when the frame is constrained into the final configuration, the sides are arcuate and bow outward slightly. The curvature of the sides allows the sides to better conform to the rounded contours of the vessel wall when the valve is deployed. In devices having a full or partial covering of material over the frame, a second method of modifying the shape is to use the material to constrain the frame in one axis. One such embodiment includes a four-sided valve with two triangular-shaped halves of material, such as SIS, where the material constrains the frame in a diamond shape. This puts the bend of the frame under stress or tension which permits better positioning within the vessel. It also allows the diagonal axis of the frame with the slit or orifice to be adjusted to the optimal length to properly size the frame for the vessel such that the leaflets open to allow sufficient flow, but do not open to such a degree that they contact the vessel wall. The potential benefits of both adding tension to the bends to bow the sides and constraining the frame into a diamond shape using the covering, can be combined in a single embodiment or employed separately.

In yet another example, the device includes a frame that in one embodiment, is formed from a single piece of wire or other material having a plurality of sides and bends each interconnecting adjacent sides. The bends can be coils, fillets, or other configurations to reduce stress and improve fatigue properties. The single piece of wire is preferably joined by an attachment mechanism, such as a piece of cannula and solder, to form a closed circumference frame. The device has a first configuration wherein the sides and bends generally lie within a single, flat plane. In an embodiment having four equal sides, the frame is folded into a second configuration where opposite bends are brought in closer proximity to one another toward one end of the device, while the other opposite ends are folded in closer proximity together toward the opposite end of the device. In the second configuration, the device becomes a self-expanding stent. In a third configuration, the device is compressed into a delivery device, such as a catheter, such that the sides are generally beside one another. While the preferred embodiment is four-sided, other polygonal shapes can be used as well. The frame can either be formed into a generally flat configuration, or into the serpentine configuration for deployment from a single or multiple sections of wire or other material. Besides rounded wire, the frame can comprise wires of other cross-sectional shapes (e.g., oval, delta, D-shape), or flat wire. Additionally, the frame can be molded from a polymer or composite material, or formed from a bioabsorbable material such as polyglycolic acid and materials with similar properties. Another method is to laser cut the frame out of a metal tube, such as stainless steel or nitinol. Still yet another method is to spot weld together, or otherwise attach, a series of separate struts that become the sides of a closed frame. In further alternative embodiments, the frame can be left with one or more open gaps that are bridged by the material stretched over the remainder of the frame. The frame can also be formed integrally with the covering, typically as a thickened or strengthened edge portion that gives the device sufficient rigidity to allow it to assume the deployed configuration in the vessel. To prevent the frame from radially expanding within the vessel beyond the point which would be considered safe or desirable, the device can be formed into the serpentine configuration and a circumferentially constraining mechanism, such as a tether, strut, sleeve, etc., placed around the device, or built into the frame, to expand or unfold during deployment of the device to limit its expansion to a given diameter, such as that which is slightly larger than the vessel into which it is placed to allow anchoring, but not permit the device to exert too great a force on the vessel wall.

In the invention, barbs are extensions of the single piece of wire or other material comprising the frame i.e., the barbs are formed as integral extensions of the frame.

In yet another example, a covering, which can be a flexible synthetic material such as DACRON, or expanded polytetrafluoroethylene (ePTFE), or a natural or collagen-based material, such as an allographic tissue (such as valvular material) or a xenogeneic implant (such as SIS), can be attached to the device with sutures or other means to partially, completely, or selectively restrict fluid flow. When the covering extends over the entire aperture of the frame, the frame formed into the second configuration functions as a vascular occlusion device that once deployed, is capable of almost immediately occluding an artery. An artificial valve, such as that used in the lower legs and feet to correct incompetent veins, can be made by covering half of the frame aperture with a triangular piece of material. The artificial valve traps retrograde blood flow and seals the lumen, while normal blood flow is permitted to travel through the device. In related embodiments, the device can be used to form a stent graft for repairing damaged or diseased vessels. In a first stent graft embodiment, a pair of covered frames or stent adaptors is used to secure a tubular graft prosthesis at either end and seal the vessel. Each stent adaptor has an opening through which the graft prosthesis is placed and an elongated barb is attached to both frames. In another stent graft embodiment, one or more frames in the second configuration are used inside a sleeve to secure the device to a vessel wall.

In yet another example, there is a barbed prosthesis, such as a stent or stent graft, in which the barb comprises a basal portion that joins the strut of the prosthesis from which the barb extends, and a stress-dispersing region located between the anchoring portion and the basal portion, usually closely adjacent to the basal portion, that is adapted to better distribute stresses and strain caused by forces acting on the barb, thus preventing their concentration at a particular point which would increase the likelihood of barb fracture.

In yet another example, the basal portion and stress-dispersing region comprises a helical coil that is wound around the barb to which it is attached. The windings of the basal portion form a mechanical attachment to which a solder joint or other bonding means is added as a second means of attachment.

In one example, the last or distal winding of the helical coil comprises the stress-dispersing region and is typically of a greater pitch than the windings of the basal portion. It also does not include solder or some other bonding means that affixes it to the strut of the prosthesis, nor does it generally contact the strut. This allows the last winding to remain flexible and thus, accommodate the forces acting upon the anchoring portion of the barb, which is embedded in the adjacent tissue. The majority of the stress load acting on the barb is distributed over the entirety of the large-radius helical bend of the winding to reduce the likelihood of fracture, rather than allowing these forces to concentrate about a single point, typically where the barb first extends from the point of union between the barb and the strut (and solder joint in this particular embodiment). In a related embodiment, the second end of the barb can comprise a second anchoring portion and stress-dispersing region extending oppositely from the basal portion and area of fixation to form a double-ended barb.

In yet another example, the basal portion of the strut is secured to the strut with a piece of cannula or similar structure that is crimped or bonded in place, such as with the illustrative solder joint. The stress-dispersing region comprises a pair of bends that facilitate lateral flexing of the barb to reduce the risk of fracture. In a related embodiment, the barb extends from the solder joint, then assumes a series of stress-dispersing bends that are proximal to the anchoring portion.

In yet another example, the stress-dispersing region of the barb comprises a coiled loop bend, U-shaped bend, or other series of bends distal to the point of attachment to add flexibility to the barb, thus reducing bending fatigue and the risk of barb fracture. The barb may include both a coiled loop bend (or other type of bend) and a free helical winding to add further flexibility.

In yet another aspect of the invention, the barb can be integrally formed with the strut, such as by laser cutting a flat sheet or cannula. It can include a stress-dispersing area comprising one or more bends and/or fillets to prevent the concentration of stress in the area immediately adjacent the union between the strut and barb.

### BRIEF DESCRIPTION OF THE DRAWINGS

FIG. 1 depicts a top view of one exemplary stent;
FIG. 2 depicts a pictorial view of the example of FIG. 1;
FIG. 3 depicts a top view and enlarged, partial cross-sectional views of a second exemplary stent;
FIG. 4 depicts a side view of the example of FIG. 3 deployed in a vessel;
FIG. 5 depicts a enlarged partial view of the example of FIG. 1,
FIG. 6 depicts a partially sectioned side view of the example of FIG. 1 inside a delivery system;
FIG. 7 depicts a top view of a third example;
FIG. 8 depicts a side view of the example of FIG. 7 deployed in a vessel;
FIGs. 9-11 depict enlarged partial views of other exemplary stents;
FIG. 12 depicts a top view of a fourth example;
FIGs. 13-14 depicts side views of the embodiment of FIG. 12;
FIG. 15 depicts a top view of a fifth example;
FIG. 16 depicts a side view of the embodiment of FIG. 15;
FIG. 17 depicts a side view of a sixth example;
FIG. 18 depicts an enlarged pictorial view of a seventh example;
FIG. 19 depicts a top view of an eighth example;
FIG. 20 depicts a top view of a first exemplary multi-leaflet intraluminal valve of the present invention;
FIG. 21 depicts a top view of a second example of a multi-leaflet intraluminal valve;
FIG. 21A depicts a partial top view of another examples;
FIG. 21B depicts a top view of another example of leaflet of the present invention;
FIGs. 22-23 depict side views of the example of FIG. 21 when deployed in a vessel;
FIGs. 24-25 depict pictorial views of the examples of FIG. 21 when deployed in a vessel;
FIG. 26-26A depict the method of attaching the covering to the example of FIG. 21;
FIG. 27 depicts a pictorial view of the basic valve of FIG. 21 upon deployment with an alternative leaflet embodiment;
FIGs. 28-31 depict top views of selected examples , made using the method shown in FIG. 28;
FIG. 32 depicts a pictorial view of an example of a stent graft that includes stent adaptors;
FIG. 33 depicts a delivery system for deploying an exemplary stent; and
FIG. 34 depicts a pictorial view of an exemplary stent having returned to the first configuration following formation into the second configuration;
FIGs. 35-36 depict top views of an exemplary three-leg valve, before and after being constrained;
FIG. 37 depicts a pictorial view of the example of FIG. 35 in the deployed configuration;
FIGs. 38-39 depict top views of four-leg valve examples, before and after being constrained;
FIG. 40 depicts a pictorial view of the example of FIG. 38 in the deployed configuration;
FIG. 41 depicts a top view of a frame formed from a sheet of material;
FIG. 41A depicts a detail view of the example of FIG. 41;
FIG. 42 depicts a top view of a third example of an intraluminal valve;
FIG. 43 depicts a pictorial view a frame example formed into a deployed configuration;
FIG. 44 depicts a top view of an example of an implantable valve having an integrally formed frame and covering;
FIG. 45 depicts a cross-sectional view taken along line 45-45 of FIG.44;
FIG. 46 depicts a cross-sectional view of a second example of valve having an integrally formed frame and covering;
FIG. 47 depicts a top view of an intraluminal valve having an open frame;
FIGs. 48-49 depict a pictorial views of intraluminal valves that include a circumferentially constraining mechanism;
FIG. 50 depicts a top view of the embodiment of FIG. 22;
FIG. 51 depicts the example of FIG. 22 having tilted following deployment within a vessel;
FIG. 52 depicts a top view of the valve in FIG. 51;
FIGs. 53-57 depict pictorial views of examples that include centering support structure comprising one or more adjoining frames or stents;
FIG. 58 depicts a side view of an example that includes centering support structure comprising a pair of lateral arms;
FIGs. 59-61 depict pictorial views of different frame examples of the basic example of FIG. 58;
FIGs. 62-62A depict pictorial views of exemplary valves that include lateral support arms and supplemental support legs;
FIG. 63-64 depict pictorial views of exemplary valves wherein the frame and centering support structure comprise a serpentine stent frame;
FIG. 65 depict a pictorial view of an example having two lateral support arms originating from each leg;
FIGs. 66-67 depict pictorial views of examples wherein the valve and centering support structure are formed from a cannula;
FIG. 68 depicts a side view of an example wherein the centering support structure comprises an expandable stent external to the valve portion;
FIG. 69 depicts a top view of an example wherein the valve and centering support structure are formed from a flat sheet of material;
FIG. 70 depicts a pictorial view of the example of FIG. 69;
FIG. 71 depicts a pictorial view of an example wherein the centering support structure includes a helical configuration;
FIG. 72 depicts a pictorial view of an example wherein the centering support structure includes an adjoining zigzag stent;
FIG. 73-74 depict pictorial and side views of an example wherein the centering support structure includes a distal projection;
FIG. 75 depicts a side view of an example wherein the valve and adjoining stent are interconnected by a sleeve of material;
FIGs. 76-79 depict tops view of examples wherein the flat square frame is formed from multiple components;
FIG. 80 depicts a side view an alternative frame of FIGs. 62-62A;
FIG. 81 depicts a flattened, view of a stent component of the example of FIG. 80;
FIG. 82 depicts a alternate example of the stent component of FIG. 81;
FIG. 83 depicts a flat wire with a line showing the pattern with which the stent with integral barbs will be cut in accordance with the invention
FIG. 84a depicts the wire of FIG. 83 after it has been cut;
FIG. 84b depicts a long, flat sheet with lines showing the patterns with which stents with integral barbs will be cut;
FIG. 85 depicts the stent wire of FIG. 84 after it has been polished;
FIG. 86a depicts one embodiment of the stent wire with integral barbs located at the midpoints between zigzag peaks;
FIG. 86b depicts another embodiment of the stent wire with integral barbs extending from alternate zigzag peaks;
FIG. 87 depicts one embodiment of a cannula which can be cut to form a stent wire;
FIG. 88 depicts another embodiment of a cannula which can be cut to form a stent wire;
FIG. 89 depicts the stent wire cut from the cannula of FIG. 88;
FIG. 90a depicts a zigzag stent with integral barbs in accordance with the present invention
FIG. 90b depicts the zigzag stent of FIG.90a, where the barbs are pointing generally transverse to the longitudinal axis;
FIG. 91 depicts a cannular endoluminal prosthesis with two stents of the present invention affixed to it;
FIG. 92 depicts an aortouni-iliac prosthesis with stents of the present invention affixed to it;
FIG. 93 depicts a side view of a prior art barb soldered to the strut of a stent;
FIG. 94 depicts a side view of an example of a stent barb having a stress-dispersing region;
FIG. 95 depicts a side view of the barb of FIG. 94 prior to attachment to the strut;
FIG. 96 depicts a side view of an example in which the barb is attached to the strut using a piece of cannula;
FIGs. 97-97b each depict a side view of an example in which the stress-dispersing region of the barb includes a coiled bend;
FIG. 98 depicts a side view of an example in which the stress-dispersing region of the barb comprises a complex bend;
FIG. 99 depicts a side view of an embodiment of the present invention in which the barb in integral with the strut of the stent; and
FIG. 100 depicts a side view of an example in which the barb includes more than one anchoring portion and associated stress-dispersing region.

### DETAILED DESCRIPTION

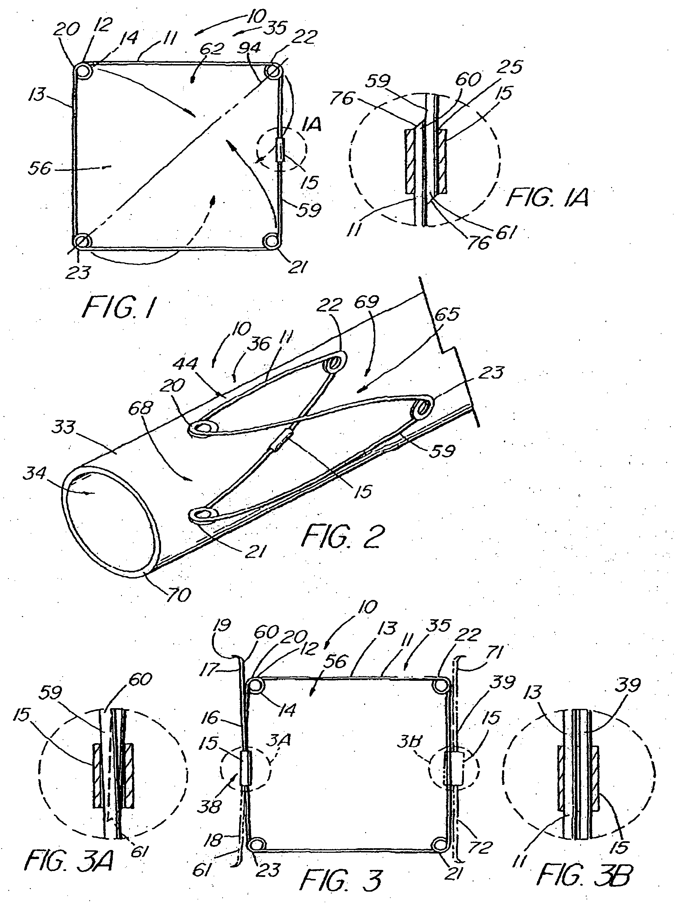

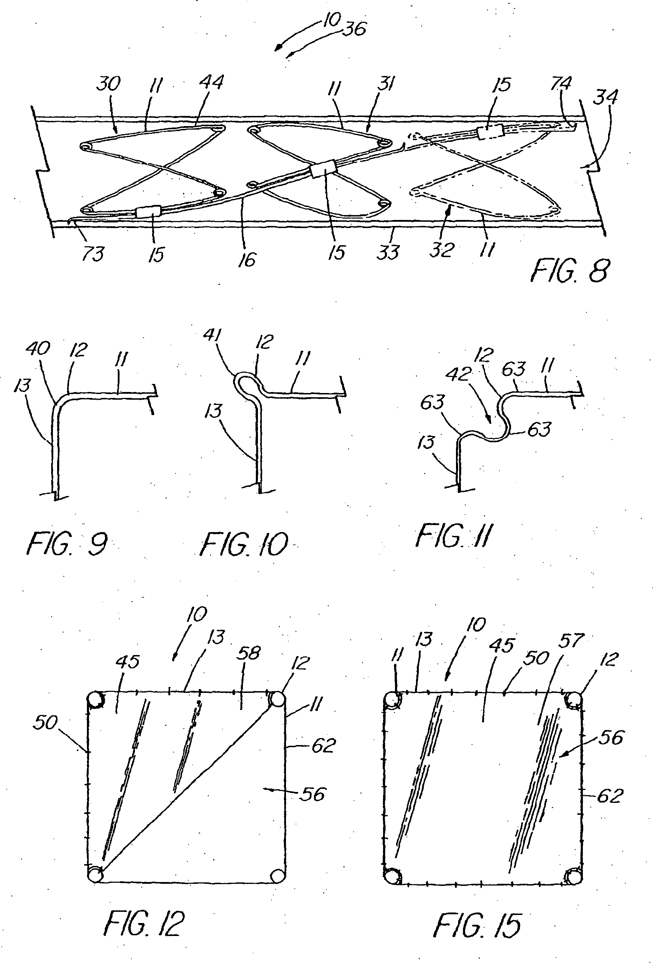

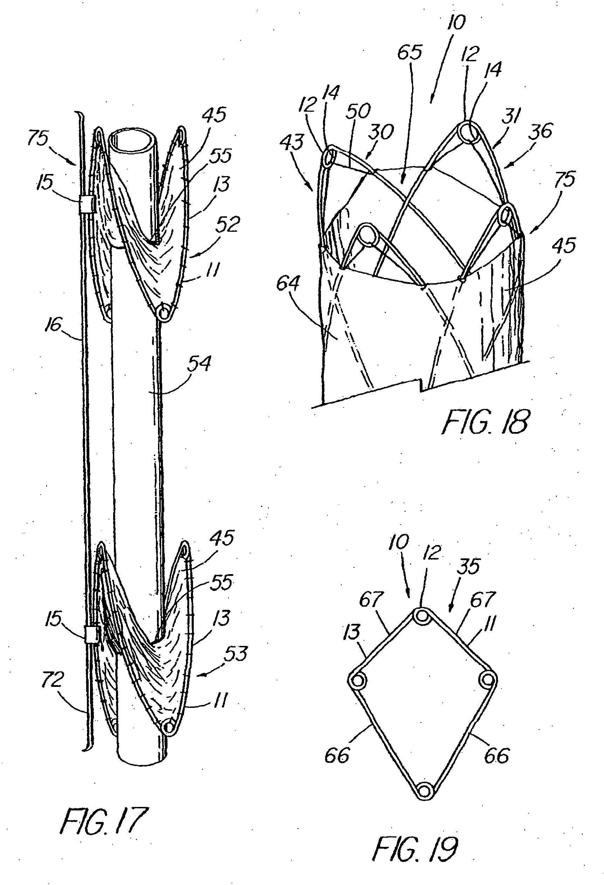

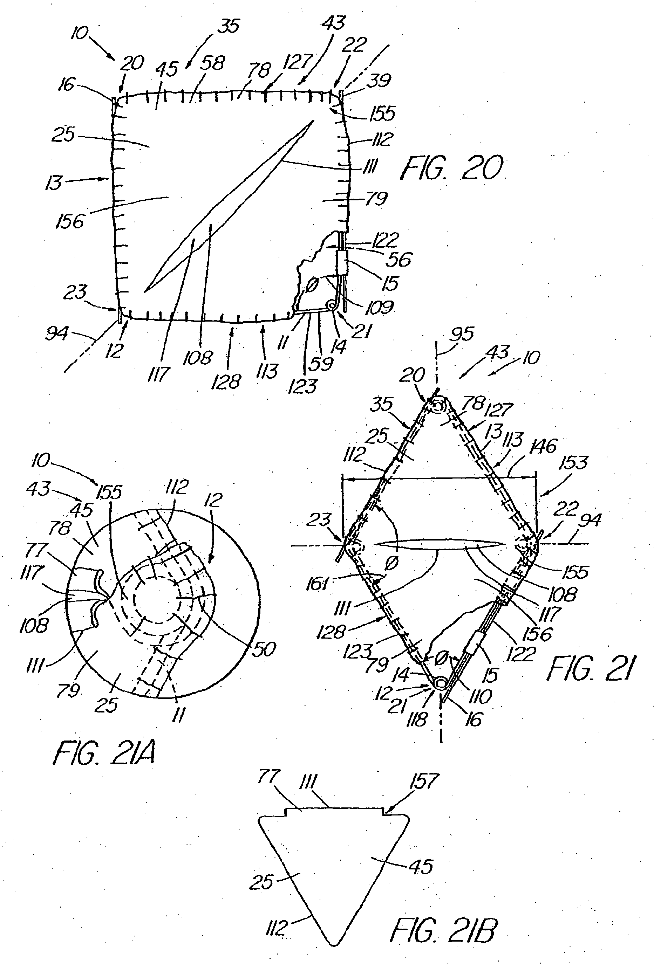

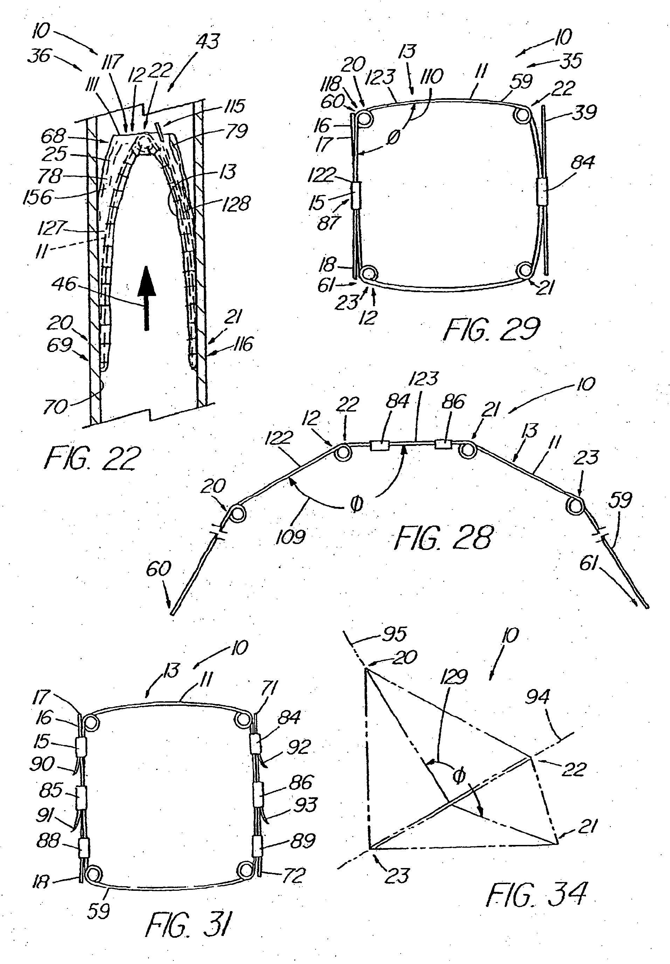

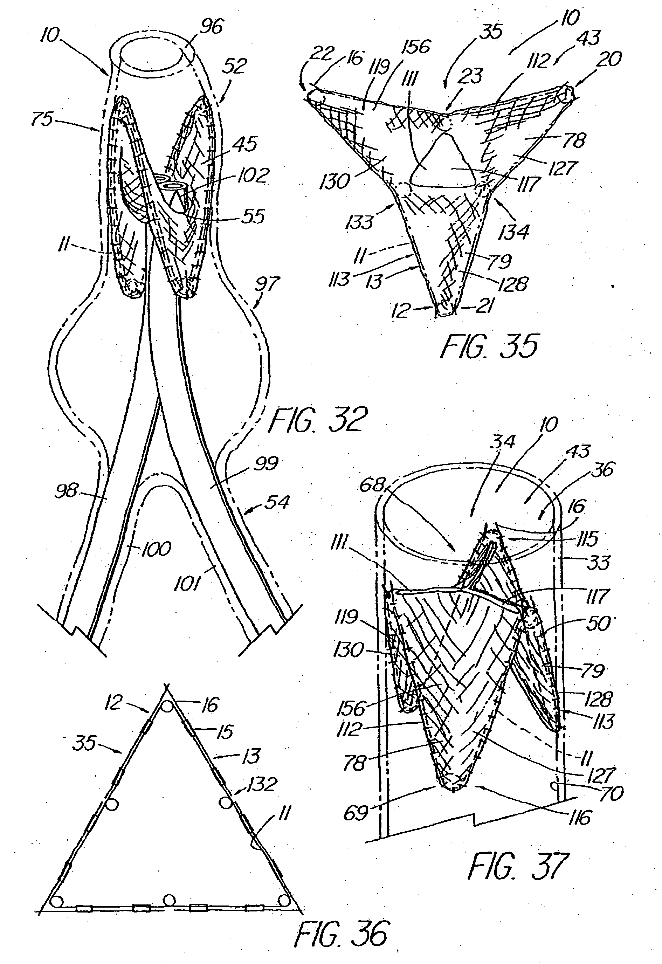

FIGs. 1-11,18-19 are directed to a basic stent frame; FIGs. 12-14 are directed to a single-leaflet valve; FIGs. 15-16 are directed to an occluder (or filter); FIG. 17 and 32 are directed to a stent adaptor for a stent graft, FIG. 20-27, 35-40, 42-50 are directed to a multi-leaf valve; and FIG. 28-31 are directed to a constrained frame which can be used to form any of the other examples.

As used herein, a 'barb' is defined as an elongate or short structure such as a straight or curvilinear wire, hook, projection, etc., typically including a distal end that includes a sharp edge and/or point, that extends outward from some portion of the prosthesis and is designed to penetrate tissue adjacent to the prosthesis, such as the walls of a vessel, to temporarily or permanently anchor the device at the location of deployment within the body of a patient. The barb can comprise the same material as the prosthesis, such as stainless steel, a superelastic alloy, polymer, etc., or of a different material. The barb is an integral part of the prosthesis. The prosthesis can be further secured mechanically, by being wound or crimped; being bonded, such as by solder, an adhesive, or welding; being fastened in a manner to allow it to slide along the strut (typically until contacting a stop). It may be advantageous in particular applications to form the stress-dispersing region in a manner to prevent significant residual stresses in the material. This can be accomplished by the use of methods or materials well known the art.

FIG. 1 depicts a top view of one example of the medical device 10 comprising a frame 11 of resilient material, preferably metal wire made of stainless steel or a superelastic alloy (e.g., nitinol). While round wire is depicted in each of the embodiments shown herein, other types, e.g., flat, square, triangular, D-shaped, delta-shaped, etc. may be used to form the frame. In the illustrative example, the frame comprises a closed circumference 62 of a single piece 59 of material that is formed into a device 10 having a plurality of sides 13 interconnected by a series of bends 12. The depicted example includes four sides 13 of approximately equal length. Alternative examples include forming a frame into any polygonal shape, for example a pentagon, hexagon, octagon, etc. One alternative example is shown in FIG. 19 that includes a four-sided frame 11 having the general shape of a kite with two adjacent longer sides 66 and two adjacent shorter sides 67. In the example of FIG. 1, the bends 12 interconnecting the sides 13 comprise a coil 14 of approximately one and a quarter turns. The coil bend produces superior bending fatigue characteristics than that of a simple bend 40, as shown in FIG. 9, when the frame is formed from stainless steel and most other standard materials. The example of FIG. 9 may be more appropriate, however, if the frame is formed from nitinol (NiTi) or other superelastic alloys, as forming certain type of bends, such as coil 14, may actually decrease fatigue life of a device of superelastic materials. Therefore, the bend 12 should be of a structure that minimizes bending fatigue. Alternative bend 12 examples include an outward-projecting fillet 41 as shown in FIG. 10, and an inward-projecting fillet 42 comprising a series of curves 63, as shown in FIG. 11. Fillets are well known in the stent art as a means to reduce stresses in bends. By having the fillet extend inward as depicted in FIG. 11, there is less potential trauma to the vessel wall.

When using stainless steel wire, the size of the wire which should be selected depends on the size of device and the application. An occlusion device, for example, preferably uses .010" wire for a 10 mm square frame, while .014" and .016" wire would be used for 20 mm and 30 mm frames, respectively. Wire that is too stiff can damage the vessel, not conform well to the vessel wall; and increase the profile of the device when loaded in the delivery system prior to deployment.

Returning to FIG. 1, the single piece 59 of material comprising the frame 11 is formed into the closed circumference 62 by securing the first and second ends 60,61 with an attachment mechanism 15 such as a piece of metal cannula. The ends 60,61 of the single piece 59 are then inserted into the cannula 15 and secured with solder 25, a weld, adhesive, or crimping to form the closed frame 11. The ends 60,61 of the single piece 59 can be joined directly without addition of a cannula 15, such as by soldering, welding, or other methods to join ends 61 and 62. Besides joining the wire, the frame could be fabricated as a single piece of material 59, by stamping or cutting the frame 11 from another sheet (e.g., with a laser), fabricating from a mold, or some similar method of producing a unitary frame.

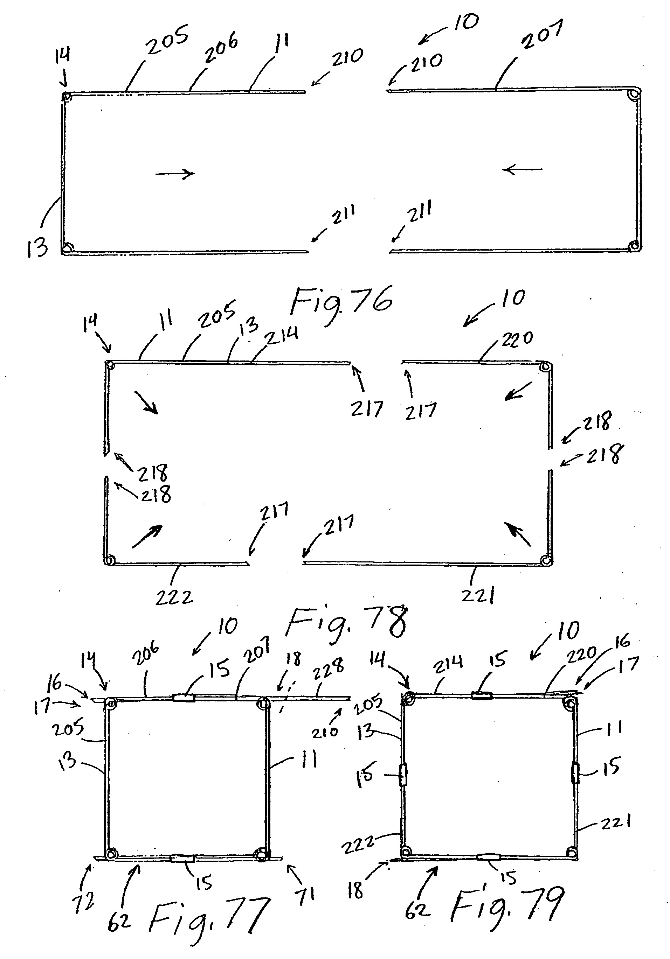

A alternate method of forming the frame 11 is depicted in FIGs. 76-79, whereby rather than one continuous length of wire being used, the frame 11 comprises a two or more sub-portions 205 that include an attachment 15 such as a weld, solder, glue, crimping with the illustrative cannula 15, or another means, or combination thereof, to form a closed circumference 62. In the example depicted in FIG. 76-77, a first and a second C-shaped sub-portion 206,207 are overlaid such that first ends 210 of the C-shaped sub-portion 206,207 extend beyond the adjoining sub-portion to form a barb 16 for anchoring the device 10 within the vessel. As shown in FIG. 77, the assembled frame 11 includes four barbs that either represent the ends 210,211 of the sub-portions 206,207, or are formed by cutting away excess material 228 from the ends, depending on how the sides 13 of the C-shaped portions are sized.

FIGs. 78-79 depict an alternative example using sub-portions 205 to assemble a closed frame, whereby there are four L-shaped sub-portions 214,220,221,222 with attachments at each of the four sides 13 that make up the closed circumference 62. In the illustrative examples only two of the ends 217 are used to form barbs 17,18; however, additional barbs can be formed by lengthening any leg of the L-shaped sub-portion 214,220,221,222 such that it extends beyond the closed circumference 62. Other configurations are possible in addition to those depicted, for example, having three sub-portions 205 or even more than four if making a frame having more than four sides.

The device 10 depicted in FIG. 1 is shown in its first configuration 35 whereby all four bends 20, 21, 22, 23 and each of the sides 13 generally lie within a single flat plane. To resiliently reshape the device 10 into a second configuration 36, shown in FIG. 2, the frame 11 of FIG. 1 is folded twice, first along one diagonal axis 94 with opposite bends 20 and 21 being brought into closer proximity, followed by opposite bends 22 and 23 being folded together and brought into closer proximity in the opposite direction. The second configuration 36, depicted in FIG. 2, has two opposite bends 20,21 oriented at the first end 68 of the device 10, while the other opposite bends 22,23 are oriented at the second end 69 of the device 10 and rotated approximately 90° with respect to bends 20 and 21 when viewed in cross-section. The medical device in the second configuration 36 can be used as a stent 44 to maintain an open lumen 34 in a vessel 33, such as a vein, artery, or duct. The bending stresses introduced to the frame 11 by the first and second folds required to form the device 10 into the second configuration 36, apply force radially outward against the vessel wall 70 to hold the device 10 in place and prevent vessel closure. Absent any significant plastic deformation occurring during folding and deployment, the device in the second configuration 36, when not with the vessel or other constraining means, will at least partially return to the first configuration 25, although some deformation can occur as depicted in FIG. 34, depending on the material used. It is possible to plastically form the stent into this configuration which represents an intermediate condition between the first configuration (which it also can obtain) and the second configuration. It is also possible to plastically deform the device 10 into the second configuration 36, such that it does not unfold when restraint is removed. This might be particularly desired if the device is made from nitinol or a superelastic alloy.

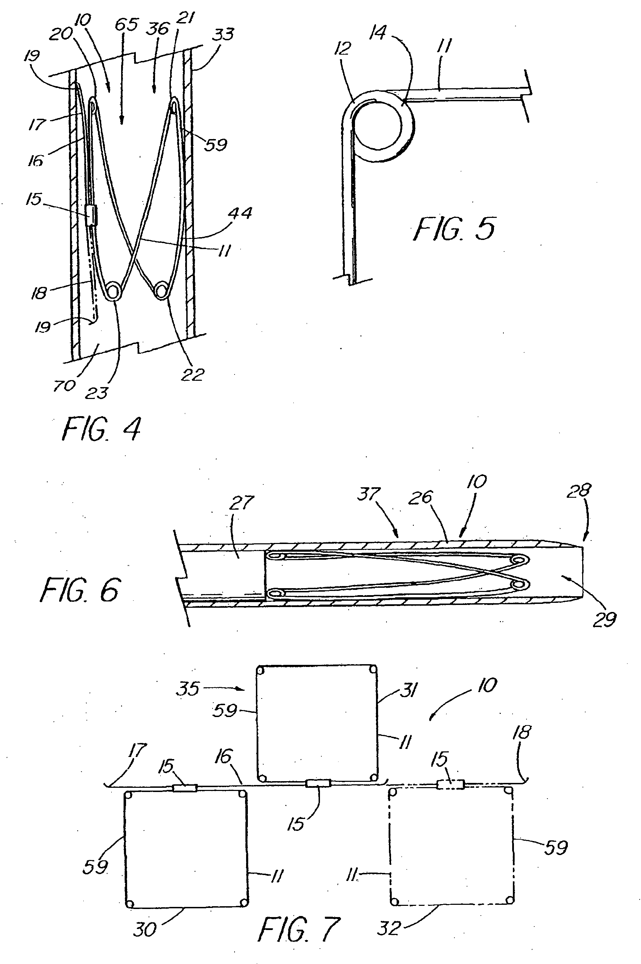

The standard method of deploying the medical device 10 in a vessel 33, depicted in FIG. 6, involves resiliently forming the frame 11 into a third configuration 37 to load into a delivery device 26, such as a catheter. In the third configuration 37 the adjacent sides 13 are generally beside each other in close proximity extending generally along the same axis. To advance and deploy the device from the distal end 28 of the delivery catheter 26, a pusher 27 is placed into the catheter lumen 29. When the device 10 is fully deployed, it assumes the second configuration 36 within the vessel as depicted in FIG. 2. The sides 13 of the frame, being made of resilient material, conform to the shape of the vessel wall 70 such that when viewed on end, the device 10 has a circular appearance when deployed in a round vessel. As a result, sides 13 are arcuate or slightly bowed out to better conform to the vessel wall.

A second example is depicted in FIG. 3 wherein one or more barbs 16 are included to anchor the device 10 following deployment. As understood, a barb can be a wire, hook, or any structure attached to the frame and so configured as to be able to anchor the device 10 within a lumen. The illustrative example includes a first barb 16 with up to three other barbs 17,71,72, indicated in dashed lines, representing alternative examples. As depicted in detail view A of FIG. 3, the barb combination 38 that comprises barbs 17 and 18, each barb is an extension of the single piece 59 of material of the frame 11 beyond the closed circumference 59. The attachment cannula 15 secures and closes the single piece 59 of material into the frame 11 as previously described, while the first and second ends 60,61 thereof, extend from the cannula 15, running generally parallel with the side 13 of the frame 11 from which they extend, each preferably terminating around or slightly beyond respective bends 20,23. To facilitate anchoring, the distal end 19 of the barb 16 in the illustrative example contains a bend or hook.

Optionally, the tip of the distal end 19 can be ground to a sharpened point for better tissue penetration. To add a third and fourth barb as shown, a double ended barb 39 comprising barbs 71 and 72 is attached to the opposite side 13 as defined by bends 21 and 22. Unlike barb combination 38, the double barb 39, as shown in detail view B of FIG. 3, comprises a piece of wire, usually the length of barb combination 38, that is separate from the single piece 59 comprising the main frame 11. It is secured to the frame by attachment mechanism 15 using the methods described for FIG. 1. FIG. 4 depicts barb 17 (and 18) engaging the vessel wall 70 while the device 10 is in the second, deployed configuration 36. While this example describes up to a four barb system, more than four can be used.

FIG. 7 depicts a top view of a third example in the first configuration 35 that includes a plurality of frames 11 attached in series. In the illustrative example, a first frame 30 and second frame 31 are attached by a barb 16 that is secured to each frame by their respective attachment mechanisms 15. The barb 16 can be a double-ended barb 39 as shown in FIG. 3 (and detail view B) that is separate from the single pieces 59 comprising frames 30 and 31, or the barb may represent a long extended end of the one of the single pieces 59 as shown in detail view A of FIG. 3. Further frames, such as third frame 32 shown in dashed lines, can be added by merely extending the length of the barb 16. FIG. 8 depicts a side view of the example of FIG. 7 in the second configuration 36 as deployed in a vessel 33.

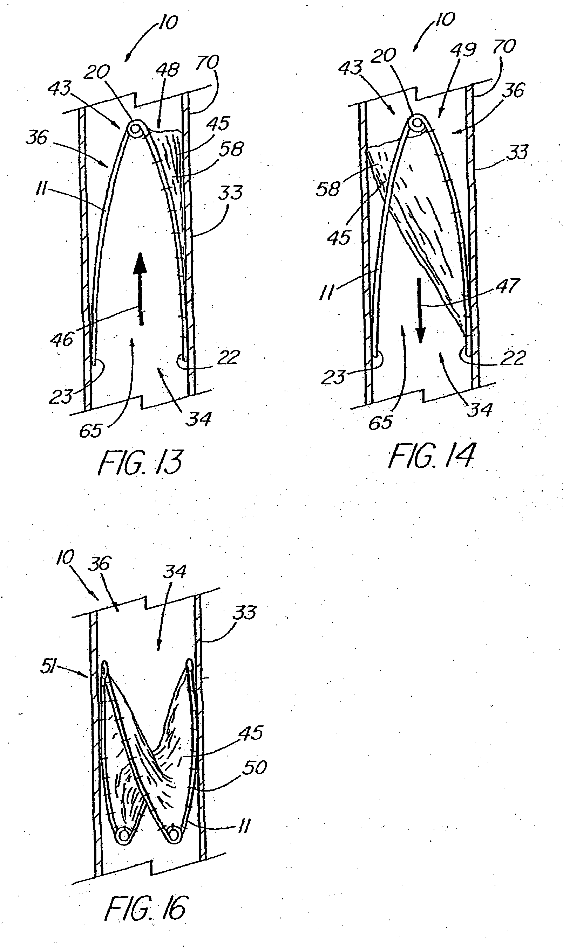

FIGs. 12-18 depict examples in which a covering 45 comprising a sheet of fabric, collagen (such as small intestinal submucosa), or other flexible material is attached to the frame 11 by means of sutures 50, adhesive, heat sealing, "weaving" together, crosslinking, or other known means. FIG. 12 depicts a top view of a fourth example while in the first configuration 35, in which the covering 45 is a partial covering 58, triangular in shape, that extends over approximately half of the aperture 56 of the frame 11. When formed into the second configuration 36 as shown in FIGs. 13-14, the device 10 can act as an artificial valve 43 such as the type used to correct valvular incompetence. FIG. 13 depicts the valve 43 in the open configuration 48. In this state, the partial covering 58 has been displaced toward the vessel wall 70 due to positive fluid pressure or flow in a first direction 46, e.g., normal venous blood flow, thereby opening a passageway 65 through the frame 11 and the lumen 34 of the vessel 33. As the muscles relax, producing flow in a second, opposite direction 47, e.g., retrograde blood flow 47, as shown in FIG. 14, the partial covering 58 acts as a normal valve by catching the backward flowing blood and closing the lumen 34 of the vessel. In the case of the artificial valve 43, the partial covering 58 is forced against the vessel wall to seal off the passageway 65, unlike a normal venous valve which has two leaflets, which are forced together during retrograde flow. Both the artificial valve 43 of the illustrative example and the normal venous valve, have a curved structure or cusp that facilitates the capture of the blood and subsequent closure. In addition to the triangular covering, other possible configurations of the partial covering 58 that result in the cupping or trapping of fluid in one direction can be used. Selecting the correct size of valve for the vessel ensures that the partial covering 58 properly seals against the vessel wall 70. If the lumen 34 of the vessel is too large for the device 10, there will be retrograde leakage around the partial covering 58.

FIG. 15 depicts a top view of a fifth example in the first configuration 35, whereby there is a full covering 57 that generally covers the entire aperture 56 of the frame 11. When the device 10 is formed into the second configuration 36, as depicted in FIG. 16, it becomes useful as an occlusion device 51 to occlude a duct or vessel, close a shunt, repair a defect, or other application where complete or substantially complete prevention of flow is desired. As an intravascular device, studies in swine have shown occlusion to occur almost immediately when deployed in an artery or the aorta with autopsy specimens showing that thrombus and fibrin which had filled the space around the device. The design permits it to be used successfully in large vessels such as the aorta. Generally, the occlusion device should have side 13 lengths that are at least around 50% or larger than the vessel diameter in which they are to be implanted.

FIGs. 17-18 depict two examples in which the device 10 functions as a stent graft 75 to repair a damaged or diseased vessel, such as due to formation of an aneurysm. FIG. 17 shows a stent graft 75 having a tubular graft prosthesis 54 that is held in place by a pair of frames 11 that function as stent adaptors 52,53. Each stent adaptor 52,53 has a covering attached to each of the frame sides 13 which includes a central opening 55 through which the graft prosthesis 54 is placed and held in place by friction or attachment to prevent migration. One method of preventing migration is placement of a stent adaptor 52,53 at each end and suturing the graft prosthesis 54 to the covering of the stent adaptors 52,53. The stent adaptors 52,53 provide a means to seal blood flow while centering the graft prosthesis in the vessel. A long double-ended barb 39 connects to each stent adaptor 52,53 and assists to further anchor the stent graft 75. In the example depicted in FIG. 18, the covering 45 comprises a outer sleeve 64 that is held in place by first and second 30,31 frames that function as stents 44 to hold and seal the sleeve 64 against a vessel wall and maintain an open passageway 65. In the illustrative example, the stents 44 are secured to the graft sleeve 64 by sutures 50 that are optionally anchored to the coils 14 of the bends 12. If the example of FIG. 18 is used in smaller vessels, a single frame 11 can be used at each end of the stent graft 75. Another stent graft 75 example is depicted in FIG. 32 for repairing a vessel defect 97, such as an aneurysm in a bifurcated vessel. The stent adaptor 52 is placed in the common vessel 96 such as the abdominal aorta. Two tubular grafts 54 are secured within an aperture 55 in the covering 45 of the frame 11 by one or more internal stent adapters 102, or another type of self-expanding stent, that bias the opening of the grafts 54 against the surrounding covering 45 to provide an adequate seal. Each leg 98,99 of the stent graft prosthesis 75 transverses the vessel defect 97 and feeds into their respective vessel branches 100,101 such the right and left common iliac arteries. As with the example of FIG. 17, a second stent adapter 53 can be used to anchor the other end of the tubular graft 54 in each vessel branch 100,101.

FIGs. 20-27 and 35-41 depict examples in which the device 10 comprises an implantable valve having multiple leaflets 25 that act together to regulate and augment the flow of fluid through a duct or vessel 33, or within the heart to treat patients with damaged or diseased heart valves. The covering 45 of each of these examples includes one or a series of partial coverings 58 that form the leaflets 25 of the valve. As with the other examples, the covering 45 may comprise a biomaterial or a synthetic material. While DACRON, expanded polytetrafluoroethylene (ePTFE), or other synthetic biocompatible materials can be used to fabricate the covering 45, a naturally occurring biomaterial, such as collagen, is highly desirable, particularly a specially derived collagen material known as an extracellular matrix (ECM), such as small intestinal submucosa (SIS). Besides SIS, examples ofECM's include pericardium, stomach submucosa, liver basement membrane, urinary bladder submucosa, tissue mucosa, and dura mater. SIS is particularly useful, and can be made in the fashion described in Badylak et al., US Patent 4,902,508; Intestinal Collagen Layer described in US Patent 5,733,337 to Carr and in 17 Nature Biotechnology 1083 (Nov. 1999); Cook et al., WIPO Publication WO 98/22158, dated 28 May 1998, which is the published application of PCT/US97/14855. Irrespective of the origin of the valve material (synthetic versus naturally occurring), the valve material can be made thicker by making multilaminate constructs, for example SIS constructs as described in US Patents 5,968,096; 5,955,110; 5,885,619; and 5,711,969. Animal data show that the SIS used in exemplary venous valves can be replaced by native tissue in as little as a month's time. In addition to xenogeneic biomaterials, such as SIS, autologous tissue can be harvested as well, for use in forming the leaflets of the valve. Additionally elastin or elastin like polypeptides (ELPs) and the like offer potential as a material to fabricate the covering or frame to form a device with exceptional biocompatibility. Another alternative would be to use allographs such as harvested native valve tissue. Such tissue is commercially available in a cryopreserved state.

To more completely discuss and understand the multi-leaflet valve 43 examples of FIGs. 20-27,35-41, it is useful to now add certain supplemental terminology which in some instances, could be applied to the examples depicted in the earlier figures. In the illustrative multi-leaflet examples, the valve 43 is divided into a plurality of legs 113, each of which further comprises a leaflet 25. To anchor, support, and provide the proper orientation of the leaflets 25, a separate or integral frame 11 is included, such as the wire frame 11 depicted in FIG. 1. Ideally, the wire used to construct the frame is made of a resilient material such as 302,304 stainless steel; however, a wide variety of other metals, polymers, or other materials are possible. It is possible for the frame to be made of the same material as the leaflets 25. One other example of a suitable frame material would be a superelastic alloy such as nitinol (NiTi). Resiliency of the frame 11, which provides radial expandability to the valve 43 when in the second configuration 36 for deployment, is not necessarily an essential property of the frame. For example, optional barbs 16 can provide the means to anchor the valve 43 after delivery, even if the valve 43 lacks sufficient expansile force to anchor itself against the vessel wall. Additionally, the frame can comprise a ductile material with the device 10 being designed to be balloon-expandable within the vessel.

Typically, when used as a valve to correct venous insufficiency in the lower extremities, the valve 43 *in situ* comprises a plurality of bends 12 of the frame, that provide the majority of the outward radial force that helps anchor the device to vessel wall 70, as depicted in FIGs. 22-27. When deployed, the frame assumes the undulating or serpentine configuration with a first series of bends 115 of the first or proximal end alternating with a second series of bends 116 of the second or distal end, with the second or distal bends 116 being located at the bottom of the valve distal to the heart and the first or proximal bends 115 being located at the top of the valve proximal to the heart. It should be understood that the valve can assume other orientations, depending on the particular clinical use, and thus, any directional labels used herein ('distal', 'top', etc.) are merely for reference purposes. The leaflet 25, which generally covers the valve leg 113 and therefore, assumes the same roughly triangular 'U' or 'V' shape of that portion of the frame 11 perimeter, includes an resilient arcuate outer edge 112 that conforms to and/or seals with the contours of the vessel wall 70, and an inner edge 111 that traverses the vessel lumen 34. The central portion or body 156 of the leaflet 25 extends inward from the vessel wall 70 and outer edge 112 in an oblique direction toward the first end 68 of the valve 43 where it terminates at the inner edge 111 thereof. The valve leaflets that come in contact with the vessel wall can also be arcuate as the supporting frame to better conform to and seal wit the vessel wall. The leaflets 25 assume a curvilinear shape when in the deployed configuration 36. The portion of the body 156 proximate the inner edge 111 is sufficiently flexible such that is can move in and out of contact with the inner edge 111 the opposite or other leaflets 25; however, the remainder of the body 156, particular that near the outer edge 112 or second end 69 of the device 10, can be made less flexible or even rigid in some instances, essentially functioning more for support, similar to the function of the frame 11, rather than to cooperate with other leaflet(s) 25. FIGs. 20-27 depict an implantable, intraluminal, vascular adapted for use as a implantable multi-leaflet valve 43 including a stent 44 or frame 11 with at least a partial covering 58. The covering comprises a first and a second valve leaflets 78,79 that at least partially seal the aperture 56 within the frame 11 while the valve 43 is in the deployed configuration 36 and forms the opening 117 or valve orifice which regulates the flow of fluid 46,47 through the valve. FIG. 20 shows the device 10 in the first, generally planar configuration 35 where the frame 111 is generally rectangular or in particular square in shape. The partial covering 58 forming the leaflets 78,79 generally extends across the entire frame 11 with the aperture 56 comprising a slit 108 that extends across the first axis 94 of the frame 11, the first axis being defined as traversing diagonally opposite bends (22 and 23 in this example) that are in line with the valve orifice 117 that forms the valve 43. The covering 45 is therefore divided into at least first and second portions (making it a partial covering 58) which define the first and second valve leaflets 78,79. To form the leaflets 78,79, a complete covering 45 can be slit open along the axis after it is affixed to the frame, or at least first and second adjacent triangular portions (partial coverings 58) can be separately attached, eliminating the need for mechanically forming a slit 108. In the example of FIG. 20, the slit 108 is made in the covering 45 such that the slit terminates a few millimeters from each of the corner bends 22,23, creating a pair of corner gaps 155, thereby eliminating two of the most likely sources of leakage around the valve 43. In the illustrative examples, the outer edge 112 of the partial covering 58 that comprises the leaflet 25 is stretched over the frame 11 comprising the valve leg 113 and sutured or otherwise attached as disclosed herein. The leaflet 25 is secured in place such that the material is fairly taut, such that when the valve 43 is situated in the vessel 33 and its diameter constrained to slightly less than the valve width 146, the leaflet 25 assumes a relatively loose configuration that gives it the ability to flex and invert its shape, depending on the direction of fluid flow. The inner edge 111 of the leaflet 25 is generally free and unattached to the frame and generally extends between the bends 22 and 23 (the bends 115 of the first end) of the valve leg 113. The inner edge 111 may be reinforced by some means, such as additional material or thin wire, that still would allow it to be sufficiently pliable to be able to seal against another leaflet 25 when retrograde flow 47 forces the leaflets 78,79 together. The leaflet 25 is sized and shaped such that the inner edge 111 of one leaflet 78 can meet or overlap with the inner edge 111 of the opposing leaflet 79 (or leaflets, e.g., 119,120), except when degree of normal, positive flow 46 is sufficient to force the leaflets 25 open to permit fluid passage therethrough.

The example of FIGs. 21-27 are configured into an elongated diamond shape 153 in the planar configuration 35 with the distance between the two bends 22,23 aligned with the valve orifice 117 and first axis 94 being less than the distance between bends 20 and 21 along the second, perpendicular axis 95. This diamond configuration 153 can be accomplished by forming the frame 11 into that particular shape, or constraining a square frame into a diamond shape 153, which will be discussed later. By configuring the valve 43 into the diamond shape 153, the valve legs 127,128 become more elongated in shape, which can help add stability when positioning the device 10 during deployment, provides more surface area to receive retrograde flow, and more closely mimics a natural venous valve. In the deployed configuration 36 of the example of FIG. 21, which is shown in FIGs. 22-25, the valve leaflets 78,79 are forced apart by the normal pulsatile blood flow 46 (FIGs. 22,24). The respective valve leaflets 78,79 naturally move back into closer proximity following the pulse of blood. Retrograde blood flow 47 forces the valve leaflets 78,79 against one another, as depicted in FIGs. 23 and 25 thereby closing off the lumen 34 of the vessel 33 and the valve orifice 117.

FIGs. 21A-21B depict examples of the valve 43 in which each leaflet 78,79 includes a flap 77 of overhanging material along the slit edge 111 to provide advantageous sealing dynamics when the valve 43 is in the deployed configuration 36 as depicted in FIGs. 22-25. The flaps 77 are typically formed by suturing two separate pieces of covering 45 material to the frame such that the inner edge 111 is extendable over the slit 108 and inner edge 111 of the adjacent leaflet 25. By overlapping with an adjacent flap 77 or leaflet 25, the flap 77 can provide additional means to help seal the valve orifice 117. Two examples of leaflets 25 with flaps 77 are shown. In FIG. 21A, the inner edge 111 is basically straight and extends over the first axis 94 of the frame 11. The flaps 77 can be cut to create a corner gap 155 that covers and seals the corner region around the bend 22,23. In the example of FIG. 21B, the flap 77 is cut such that there is a notch 157 in the leaflet where the leaflet meets the corner bends 22,23. While these flaps 77 may provide benefit in certain examples, the optional flaps 77 shown in FIG. 21 are not necessary to provide a good seal against backflow 47 if the valve 43 and leaflets 25 are properly sized and configured.

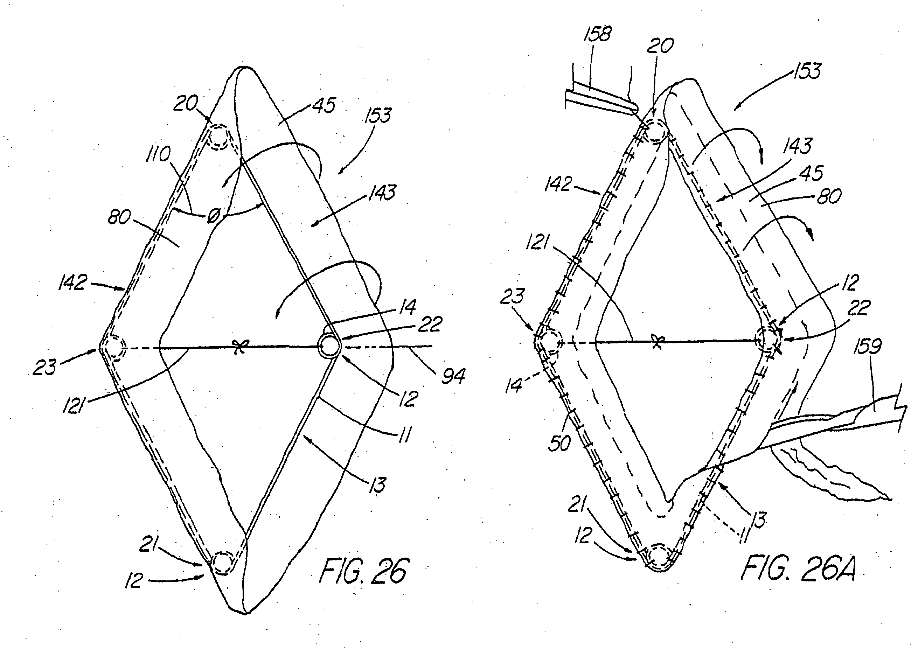

FIGs. 26-26A depict one method of affixing a covering 45 comprising a biomaterial, such as SIS, to the frame 11 which has been constrained using a temporary constraining mechanism 121, such as a suture, to achieve the desired frame configuration. As shown in FIG. 26, the covering 45 is cut larger than the frame 11 such that there is an overhang 80 of material therearound, e.g., 5-10 mm. The frame 11 is centered over the covering 45 and the overhang 80 is then folded over from one long side 142, with the other long side 143 subsequently being folded over the first. As shown in FIG. 26A, the covering 45 is sutured to the frame along one side 142, typically using forceps 158 and needle, thereby enclosing the frame 11 and the coiled eyelet 14 with the overhang 80 along side 142. The covering 45 is sutured to the frame with resorbable or non-resorbable sutures 50 or some other suitable method of attaching two layers of biomaterials can be used. In the case of SIS, a single ply sheet, usually about 0.1 mm thick, is used in the hydrated condition. In the illustrative examples, 7-0 Prolene suture is used, forming a knot at one bend (e.g., bend 20), then continuing to the next bend (e.g., 22) with a running suture 50, penetrating the layers of SIS around the frame at about 1-2 mm intervals with loops formed to hold the suture 50 in place. When the next coil turn 14 is reached, several knots are formed therethrough, and the running suture 50 continues to the next coil turn 14. If barbs are present, such as shown in the example of FIG. 21, the suture 50 is kept inside of the barbs 16 located about each coil turn 14. In the illustrative example, the covering 45 is affixed to the frame 11 such that one side of the overhang 80 is not sutured over the other side in order to maintain the free edge of the overhang 80, although the alternative condition would be an acceptably example. Alternative attachment methods include, but are not limited to, use of a biological adhesive, a cross-linking agent, heat welding, crimping, and pressure welding. For synthetic coverings, other similar methods of joining or attaching materials are available which are known in the medical arts. The covering 45, whether made from a biomaterial or synthetic material, can be altered in ways that improve its function, for example, by applying a coating of pharmacologically active materials such as heparin or cytokines, providing a thin external cellular layer, e.g., endothelial cells, or adding a hydrophilic material or other treatment to change the surface properties.

Once the covering 45 has been sutured into place or otherwise attached to the frame, the overhang 80 is folded back away from the frame, as shown on the second side 143 of the frame of FIG. 26A, and part of the excess overhang 80 is trimmed away with a scalpel 159 or other cutting instrument to leave a 2-4 mm skirt around the frame 11. The overhang 80 or skirt provides a free edge of SIS (or material with similar remodeling properties) to help encourage more rapid cell ingrowth from the vessel wall, such that the SIS replaces native tissue as quickly as possible. An unattached edge of the overhang 80 can also form a corner flap 81 or pocket as depicted in FIG. 27. This corner flap 81 can serve to catch retrograde blood flow 47 to provide a better seal between the device 10 and the vessel wall 70 as well as providing an improved substrate for ingrowth of native intimal tissue from the vessel 33, if made of SIS or another material with remodeling properties.

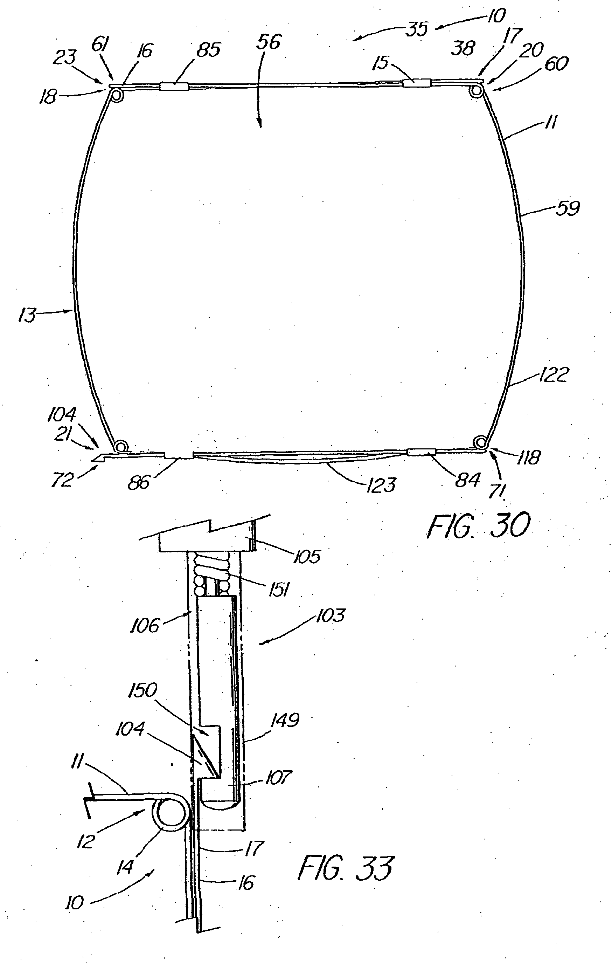

Referring now to FIGs. 28-31, the frame 11 used to form the valve 43 examples, e.g., FIGs. 20-27, that are placed in the legs or other deep veins as replacement for incompetent venous valves, is sized according to the size of the target vessel. For example, a typical venous valve might be made of .0075" 304 stainless steel mandrel wire with an attachment mechanism 15 comprising 23 to 24 gauge thin-wall stainless steel cannula or other tubing. Larger wire (e.g., 0.01") and attachment cannula 15 are typically used for valves 43 of the larger diameter (greater than 15 mm). Selection of the attachment cannula 15 depends on competing factors. For example, use of larger gauge attachment cannula 15 results in a slightly increased device 10 profile, yet it includes additional room for flux when the attachment mechanism 15 is soldered over the continuous wire 59 comprising the frame 11. FIG. 30 best depicts an uncovered frame 11 used to form a venous valve 43, wherein the length of the sides 13 typically range from about 15 to 25 mm. For larger frames, heavier gauge wire is typically used. For example, 25 mm frames might use 0.01" wire, with larger diameter examples such as stent occluders used for femoral bypass or stent adaptors, such as shown in FIGs. 17 and 32, requiring an even heavier gauge. The appropriate gauge or thickness of the frame wire also depends on the type of alloy or material used. As previously disclosed, the frame is typically formed in a generally flat configuration and then manipulated into its characteristic serpentine configuration and loaded into a delivery system. Therefore, the frame usually will tend to reassume the first or generally flat configuration if the restraint of the delivery system or vessel is removed. Deformation of the frame 11 can occur after it has been manipulated into the second configuration, however, such that it no longer will lie completely flat, as depicted in FIG. 34. This angle of deformation 129, which varies depending on the frame thickness and material used, generally does not compromise the function of the device 10, which can be reconfigured into the serpentine configuration (of the second, deployed configurations) without loss of function.

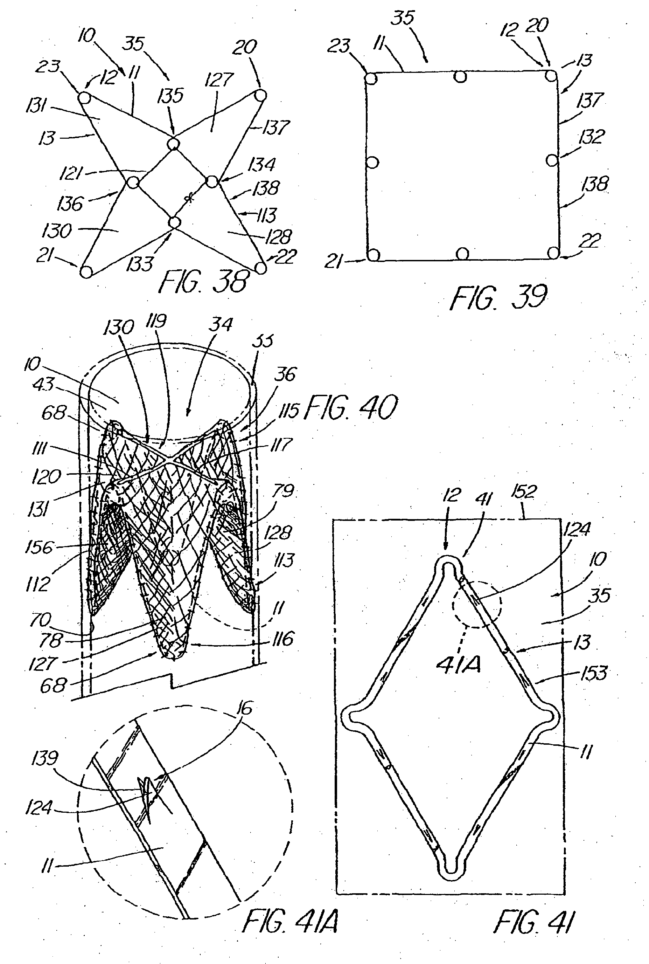

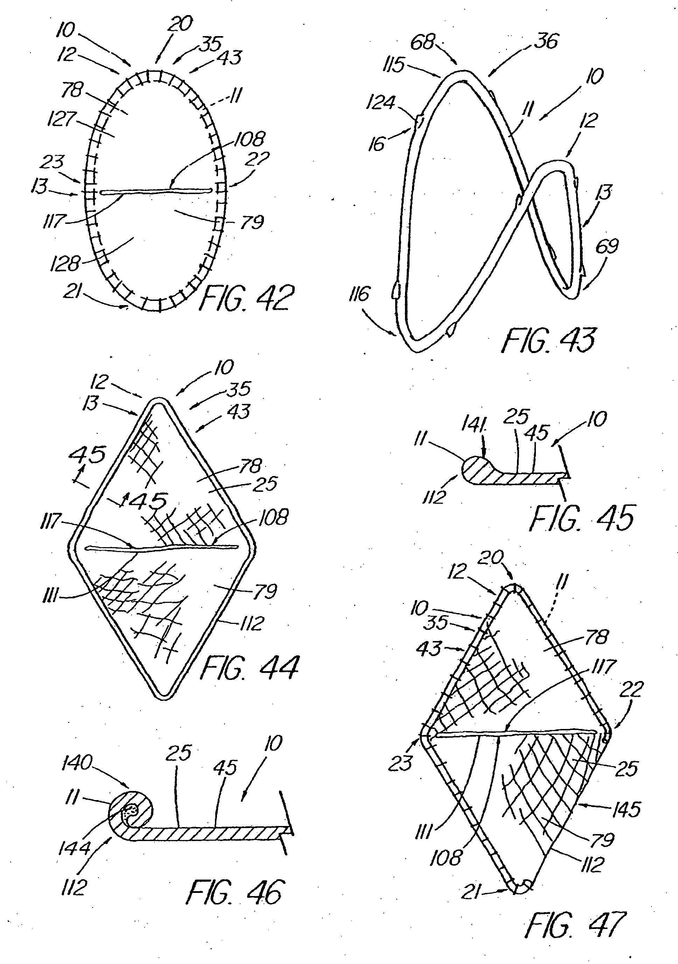

Frame 11 can be made either by forming a series of bends in a length of straight wire and attaching the wire to itself, as previously discussed, to form a closed configuration, or the frame 11 can be formed in the deployment (second) configuration 35 as depicted in FIGs. 41-41A by cutting it out of a flat sheet 152 of material, e.g., stainless steel or nitinol. Further finishing procedures can then be performed after it has been cut or formed, such as polishing, eliminating sharp edges, adding surface treatments or coatings, etc. In addition to metal, the frame 11 can comprise one or more polymers, composite materials, or other non-metallic materials such as collagen with the frame either being cut from a thin sheet of the material, or molded into the deployment configuration 36 as depicted in FIG. 43. Unlike the majority of the depicted examples, the frame 11 of FIG. 43 does not naturally assume a flattened configuration 35 when the device 10 is unconstrained by the vessel or delivery system.

The illustrative examples of FIGs. 41-41A and 43 include integral barbs 124 that extend from the frame 11, which being formed as a closed frame, does not have free ends 60,61 that can be used to serve as barbs 16 as depicted in FIG. 3 and other examples. FIGs. 41-41A depict a series of integral barbs 124 comprising V-shaped cuts 139 transversing the thickness of the flat metal frame 11, which are bent outward to form the barb 16. In the embodiment of FIG. 43, the integral barbs 124 are formed along with the frame 11 with two extending from the frame at either side of each bend 12. These integral barbs 124 can be designed into the mold if the frame 11 is formed out of a polymer material. The number, arrangement, and configuration of the integral barbs 124 are generally not critical and can vary according to design preference and the clinical use of the device. The barbs 16 may or may not penetrate the covering, depending on their design and other factors, including the thickness and type of covering used.

While the frame of FIG. 43 can be formed from a variety of medical grade polymers having properties that permit the frame to function as a supporting structure for the valve leaflets 78,79, it should be noted that for some uses, it may be desirable to form the frame 11 from a material that can be degraded and adsorbed by the body over time to advantageously eliminate a frame structure can would remain in the vessel as a foreign body and that could possibly fracture and/or cause perforation of the vessel wall. A number of bioabsorbable homopolymers, copolymers, or blends of bioabsorbable polymers are known in the medical arts. These include, but are not necessarily limited to, poly-alpha hydroxy acids such as polylactic acid, polylactide, polyglycolic acid, or polyglycolide; trimethylene carbonate; polycaprolactone; poly-beta hydroxy acids such as polyhydroxybutyrate or polyhydroxyvalerate; or other polymers such as polyphosphazines, polyorganophosphazines, polyanhydrides, polyesteramides, polyorthoesters, polyethylene oxide, polyester-ethers (e.g., polydioxanone) or polyamino acids (e.g., poly-L-glutamic acid or poly-L-lysine). There are also a number of naturally derived bioabsorbable polymers that may be suitable, including modified polysaccharides such as cellulose, chitin, and dextran or modified proteins such as fibrin and casein.

FIGs. 44-46 depict two exemplary examples in which the frame 11 is integral with the covering 45. In the example of FIG. 44, the valve 43 is formed as a single piece of material, such as a flexible polymeric or collagen-based material, whereby there is a thin, compliant central portion comprising the covering 45 or leaflets 78,79, and a thickened edge 141 portion that comprises the frame 11. The valve 43, shown in the generally flat configuration 35, can be also formed into the deployment configuration 36 (see FIG. 43). Optionally, the material of the frame 11 portion can be subjected to treatments or processes that add rigidity or other desired characteristics that permit the frame to better support the covering 45 portion or anchor the device 10 to the vessel wall. As with the example of FIG. 43, optional integral barbs 124 can be included along the frame 11. In addition to forming a thickened edge 141 to serve as the frame 11, other layers of different materials can be laminated to or blended with the edge portion to provide the desired properties. As another alternative to the thickened edge 141 portion of FIGs. 44-45, the outside edge 112 of the covering 45 can be folded over itself to form a rolled edge 140 (FIG. 46) that adds rigidity to serve as a frame 11. The rolled edge 140 can be held in placed with a glue, resin, or similar bonding agent 144. For example, the covering 45 and rolled edge 140 can comprise a sheet of SIS with a bonding agent 144 such as collagen glue or other bioabsorbable material used to secure the rolled portion and after hardening, to add the necessary degree of rigidity for the valve 43 (or occluder, filter, stent adaptor, etc.) to assume the deployment configuration within the vessel. Excess of the bonding agent 144 can be fashioned to structural elements that can serve to help anchor the device 10 within the vessel. It is also possible to eliminate a discernable frame 11 by changing the material or material properties along the outer edge 112 of the leaflets, by adding or incorporating one or more different material or agents along the outer edge 112 of covering 45 such that the stiffness and/or resiliency increased, thereby allowing the frame to hold a desired shape during deployment, while still allowing the adjacent covering material to be sufficiently flexible to function as a leaflet 25. If the illustrative valve 43 lacks the radial expandability to anchor itself to the vessel wall, it may be mounted on a balloon to expand the valve 43 and anchor the barbs, if present, into the vessel wall.

The illustrative examples generally include a closed frame 11 to give the device 10 its form. FIG. 47 depicts an example in which the frame 11 portion is not a closed structure. Rather, a portion of the covering 45 used to span a gap 145 in the frame such that a portion of the outside edge 112 (of leaflet 79 in this example) is unsupported therealong. The length of the gaps 145 and their distribution can vary as long as the frame 11 is still able to fulfill its role to support and define the shape of the valve 43 or device 10.

FIGs. 21-31 depict various examples in which the bends 20,21,22,23 are placed in a resiliently tensioned or stressed state after being initially formed such that the bends were not under tension. The term 'tension', as used herein, is meant to describe generally a forced applied to a resilient material or structure against the natural tendency of the material or structure, whether or not the force is in fact tensile, compression, or torsional. Further incremental forces applied will generally encounter greater resistance than would otherwise be exhibited by the material or structure, such as a compression spring, which exerts a force (resilience) resisting compression proportional to the distance the spring has already been compressed. The addition of tension to one or more bends 12 of the device frame 11 can alter the properties of the frame 11 and result in improved sealing characteristics or the ability of the device 10 to impinge upon the vessel wall 70 to prevent migration or shifting. In the illustrative examples, the coil turn 14 is formed as previously disclosed whereby each bend 12 is in a untensioned state with the adjacent sides 13 having an initial angle after formation of the bend 12. For example, in the example of FIG. 20, the initial angle 109 after the bends are formed and the final angle 110 after the frame 11 is assembled are both approximately 90°. Therefore, the bends 12 of the example of FIG. 20 are not placed under any significant degree of tension. In the examples of FIGs. 21-31, the frame is restrained to permanently place the bends 12 under tension such that the angle between the sides 122,123 adjacent to the bend 12 is increased or decreased by some method of manipulation to produce a resiliently tensioned bend 118 (FIGs. 26 and 29) having a final angle 110 different than the initial angle 109 (e.g., FIG. 28).

Referring particularly to FIGs. 21-28, the covering 45 (including a full or a partial covering 58) can be attached to the frame 11 of the valve 43 or other disclosed example, to constrain a generally untensioned square frame 11 (such as in FIG. 1) and subsequently form an altered shape 82, such as a diamond 153, in which the distance between bends 20 and 21 is lengthened and the distance between bends 22 and 23 is shortened. By way of example, and using FIG. 21 as reference, the angle 110 measured between the adjacent sides 13 from bends 20 and 21 might decrease to 70-80° with a increase in the corresponding angles 161 measured at bends 22 and 23 to 100-110°. This manipulation of the frame 11 shape serves to add tension in each of the bends, which allows better positioning of the device 10 against the vessel wall 70 while in the deployed configuration, as shown in FIGs. 22-25. Additionally, constraining the frame 11 along the first axis 94 of the slit 108 allows that distance 146 to be adjusted to provide the optimum size for the vessel 33 into which the valve 43 is to be implanted. Assuming a resilient frame 11 is being used that makes the valve 43 radially expandable, it would normally be preferential to slightly oversize the valve 43 along at the width 146 of the frame 11 (along first axis 94) when the valve 43 is in the generally flattened configuration 35, thereby causing the leaflets 78,79 to relax slightly when the valve 43 is in the deployed configuration 36 and being constrained slightly by the vessel 33. The proper length of the constrained frame 11 as measured diagonally between bends 22 and 23 is calculated such that the leaflets 78,79 open by an effective amount in the presence of blood flow 46 that most closely mimics that found in a normal functioning valve.

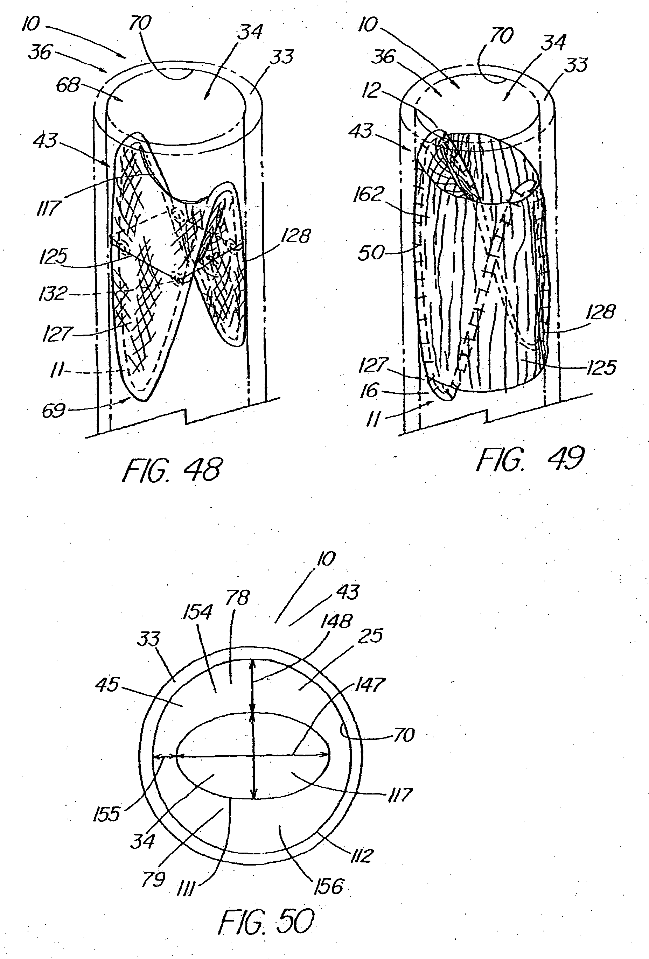

Dog studies by Karino and Motomiya (Thrombosis Research 36: 245-257) have demonstrated that there is about a 60 to 70% constriction of blood flow through the natural valve. In the disclosed valve 43, the leaflets 25 should ideally span about 30-60% of the vessel 33 diameter across. If it is much less than 30%, blood flow 46 may be impeded to an unacceptable degree, while if the leaflets 78,79 are allowed to fully open, they can adhere to the vessel wall 70 and therefore, not close properly in the presence of retrograde flow 47. The frame 11 can be formed or constrained such that the distance 146 between points 22,23 lies between πr, which would allow the valve to open to the full extent that the vessel allows, and 2r in which the valve 43 is stretched tight across the frame 11 and is very limited in the amount of blood that will allow to pass through. To give the leaflets the flexibility and compliance to open to permit flow and then close to seal against backflow, the slit axis distance 146 of the valve 43 should be oversized with respect to the diameter of the vessel into which it is to be placed. Constraining the valve 43 along the first axis 94 such that it sized a few mm larger than the lower extreme (2r) or a few mm larger than the upper extreme (πr), not only allows the leaflets to function in a more optimal manner, but also allows the valve 43 to safely and effectively impinge on the vessel wall to seal and reduce the possibility of migration. The ideal amount of oversize is largely dependent on the size and diameter of the frame 11 prior to resizing. FIG. 50 depicts a schematic top view of the valve of FIG. 22 showing the length 147 of the orifice, the width 148 of the orifice, the portion 154 of the vessel occluded by a leaflet 25, and the corner gaps 155 than exist between each lateral edge 156 of the valve orifice 117 and the outer edge 112 of the leaflet 25 (or the frame 11). The following formula can be to approximate the elliptic circumference (C) of the valve orifice 117, where a=one half the length 147 of the orifice, and b=one half the width 148 of the orifice 117:

Assuming that we wish to size the valve 43 to produce an orifice 117 that opens approximately 30-60% of the vessel lumen 34 (with the occluded portions 154 comprising 40-70% of the same), the preceding formula can be used to determine the amount of oversize that produces the desired characteristics. The amount of oversize (valve width 146 in the flat configuration minus the diameter of the vessel lumen 34) would generally range from 1-2 mm for smaller valves (those placed in 8-9 mm vessels) up to 3-4 mm for valves intended for larger vessels (17-21 mm). For example, a valve intended for a 14 mm vessel should ideally have a 2-3 mm oversize if the range of 30-60% opening is to be maintained. If the frame 11 of a valve 43 having 20 mm sides is constrained such that the distance between bends 22 and 23 is adjusted to approximately 16 mm, the valve 43 opens approximately 43%, which is well within the most desired range. If constrained to 17 mm, the valve 43 is able to open up to approximately 55% of the vessel diameter. In contrast, oversizing the valve 43 by 6 mm, produces a large orifice 117 of 83% which lies outside the target range, although it would certainly produce a valve 43 capable of opening and closing in response to fluid flow 46,47. To produce a valve 43 in which the valve width in the generally flattened configuration 35 is 17-18 mm, which would be a valve 43 sized to accommodate a 14-15 mm vessel, the 20 mm frame 11 should be constrained such that the distance between bends 22 and 23 is 15 mm prior to addition of the covering 45, if a compliant material such as SIS is used. As depicted in FIG. 26, the frame 11 is constrained across the first axis 94 using a temporary constraining mechanism 121, such as by tying a suture through the coil turns 14 of bends 22 and 23 to pull them toward one another until a distance of 15 mm is reached. After the covering 45 has been attached, such as by the method previously disclosed, the temporary constraining suture 121 is cut, which results in a slight expansion in the width of the frame 11 as the SIS stretches under the tension of the constrained frame, resulting in the desired final width of 17-18 mm. The amount of expansion varies with the compliance of the particular covering 45 as well as the resiliency of the frame 11. Although the desired final width 146 of the constrained frame 11 can result from a relatively wide range of initial frame 11 sizes, depending on how much the frame is constrained, generally, larger sized frames (e.g., sides measuring about 25 mm) are most suitable for larger vessels (e.g., 16-21 mm in diameter), while smaller frames (e.g., 15 mm) are most suitable for smaller diameter vessels (e.g., 8-9 mm). While this range represents the most common sizes used for correcting venous valve insufficiency in the lower legs, valves 43 can be made in a much larger range of sizes to treat veins or other vessels elsewhere in the body.

FIGs. 28-31 depict another example in which a open frame 11, such as depicted in FIG. 28, is assembled into a square frame (FIGs. 29-31) such the bends 12 are put under tension. The resiliently tensioned bends 118 in the assembled device (as shown in FIGs. 29-31) result from the initial angle 109 formed in wire frame 11 before being assembled into a closed circumference 62 (FIG. 28), being greater than the final angle 110. To form the example of FIG. 1, for example, the wire is wrapped around a pin to form the coil turns 14 with the sides 13 generally lying about 90° with respect to one another. The attachment mechanism 15 then secures and closes the frame 11 to form the final square shape. In the examples of FIGs. 28-31, the first angle 109 is made approximately 150°, rather than 90°, which is the desired final angle 110. While the wire is not under stress after the bends 12 are initially formed, the bends 12 and sides 13 are stressed when the device 10 is constrained during assembly to form the four-sided, generally square shape. In particular reference to FIG. 30, the sides 122,123 adjacent to a resiliently tensioned bend 118 becomes somewhat deformed when the bend 12 is put under stress, generally assuming a bowed shape between the adjacent bends. By creating this 'rounded square' with tensioned or stressed bends 118, the sides 13 of the frame 11 are able to better conform to the rounded vessel wall 70 than would a side 13 that is initially straight prior to deployment. Additionally, by rounding the distal bends 116 of the valve legs 113, it may also reduce the potential for the valve legs 113 to cause trauma to the vessel 33 as they continue to exert force thereupon.

An additional method of constraining the valve 43, or similar type device 10 (e.g., occluder, filter, stent, stent adaptor), is depicted in FIG. 48 in which a circumferentially constraining mechanism 125, is added to at least partially encircle the frame 11 while it is in both the delivery configuration 37 (FIG. 6) and the deployed configuration 36 such that the device 10 is limited in its ability to radially expand. Once the device reaches its maximal radial expansion, the outward force the device 10 places on the vessel wall 70 is eliminated, thereby reducing potential damage thereto (e.g., from an improperly sized valve), such as tissue erosion possibly resulting in eventual perforation of the vessel 33. In the illustrative example, the circumferentially constraining mechanism 125 comprises a suture that is affixed to and completely encircles the frame 11 to limit the outward expansion of the valve legs 127,128. The sides 13 of the valve legs 127,128 include an intermediate coil turn 126, also illustrated in FIG. 39 fulfilling a different function, that provides an effective attachment point through which to feed and/or affix the suture restraint 125. In the illustrative example, the suture restraint 125 is in a relaxed state when the device 10 is loaded in the delivery system. Then, as the device 10 is deployed, it expands within the vessel 33 until it is constrained by the suture restraint 125 if the device 10 has been properly sized such that vessel 33 does not provide constraining forces sufficient to prevent the device 10 from fully expanding to its predetermined maximum diameter. If the device is undersized for the diameter of the vessel, it may be subject to migration due to insufficient expansion. The illustrative example is merely exemplary of the numerous available circumferentially constraining mechanisms 125. It is not necessary that the circumferentially constraining mechanism 125 completely encircle the device 10. For example, short pieces of suture or another type of tethering mechanism, such as a section of webbing or other material, can be affixed between the sides of the valve legs to limit their expansion, or the frame can include an integral circumferentially constraining mechanism, such as an expandable strut formed as part of the frame. The strut would unfold as the frame radially expands and limits how far the sides of the valve leg to which is attached, can spread apart relative to each other, thereby limiting the outward radial force from the device against the vessel wall.

Another possibility is for circumferentially constraining mechanism 125 to comprise a sleeve 162 of flexible material, such as SIS around the valve 43, as depicted in FIG. 49, which is of a diameter appropriate for deployment within the target vessel 33 (typically, being slightly larger than the target vessel diameter) that allows the valve to anchor thereto. The sleeve 162 could be affixed to the frame 11 with sutures 50 or by some other means as the valve 43 is held in a collapsed condition prior to loading the device 10, including the sleeve 162, into a delivery system. The sleeve 162 enclosed the length of the valve 43, or the bends 12 and barbs 16 can be left uncovered, as shown. To reduce resiliency of the sleeve 162, tethers and other types of circumferentially constraining mechanism 125 can be used in combination with the sleeve 162 to limit radial expandability of the valve 43. It should be noted that if the circumferentially constraining mechanism 125 itself is a resilient member, it will only serve to reduce the outward force of the device 10 against the vessel wall 70 until maximum expansion is reached.

FIGs. 30-31 depict alternative methods of forming the frame 11 and attaching barbs thereto. In the example shown in FIG. 30, attachment mechanisms 15,85 and 84,86, per side rather than a single cannula as shown in previous examples, such as FIG. 29. Rather than placing the attachment mechanisms 15 at the point 87 where the respective ends 60,61 of the wire frame 11 cross to form the square shape, two attachment mechanisms 15,85 are placed on either side of the cross point 87. Having an additional attachment mechanism 84,85,86 on a side 13 provides better fixation of the frame with little additional metal and helps prevent twisting of the frame 11. On the opposite side which contains the double ended barb 39, the double attachment mechanisms 84, 86 arrangement provides a similar function. In the example of FIG. 31, three attachment mechanisms 15,85,88 and 84,86,89, are used per side which provide better fixation of the frame 11 as well as serving as attachment points for including supplemental barbs 90,91,92,93 to provide a more secure anchoring of the device 10 to the vessel wall 70. The illustrative barbs 16 are typically configured such that they extend only a short distance (less than 1-2 mm) beyond the bends 12; however, the barbs 16 can be made virtually any practical length, such as extending them more than 1 cm beyond the bends 12 to and in stabilizing the device 10 upon deployment such that it does not shift laterally and end up being cockeyed within the vessel. To assist in this function, the barbs can be shaped accordingly, rather than be limited to a substantially straight configuration.

FIGs. 35-40 depict multi-leaflet valves 43 haying three or four valve legs 113 and leaflets 25. The addition of additional leaflets reduces the load produced by the fluid column upon each individual leaflet 25. This in turn, puts less stress upon the sutures or attachment points of the covering 45, thereby allowing the valve 43 to function under higher pressures than would otherwise be possible. For example, these valves 43 could prove advantageous for use on the arterial side, such as to augment pulmonary valves, or within the heart itself, where pressures exerted on the leaflets can be significantly higher than normally found on the venous side. FIG. 35 depicts a valve 43 which in the generally flattened configuration 35, has a three legs 127,128,130 that lie approximately 120° with respect to one another. The respective leaflets are arranged such that the inner edges 111 thereof, define a triangular-shaped valve orifice 117. When the illustrative valve 43 is placed in the vessel 33 for which it has been properly sized, as depicted in FIG. 37, the leaflets 78,79,119 are able to close against one another to seal the valve. The concept of adding additional legs 113 to distribute the load over a larger number of attachment points 50 (e.g., sutures) and add positional stability to the device 10, can be applied to occluders and stent adaptors as well.

One method of forming the example of FIG. 35, involves constructing a triangular-shaped frame 11, as shown in FIG. 36, that includes an intermediate coiled eyelet 132 formed at the midpoint of each of the three sides 13. A temporary constraining suture 121, such as that shown in FIG. 38, is threaded through each of the intermediate eyelets 132, drawing them inward to form three additional bends 133,134,135 forming three legs 127,128,130 of a desired shape (FIG. 35), depending how tightly the constraining suture 121 is drawn. At this point, the covering 45 is attached to the frame 11, either as three separate leaflets 78,79,119, or a single piece through which the triangular-shaped valve orifice 117 is formed. After the covering 45 has been secured to the frame 11, the constraining suture 121 is cut and removed. As depicted, the barbs 16 are affixed to the triangular shaped frame of FIG. 36, two per side, such that they terminate on either side of intermediate eyelet 132. Thus, when the intermediate eyelets 132 are drawn inward to create six sides 13, each includes a barb 16.

The example of FIGs. 38-40, which includes four legs 127,128,130,131, is formed in a similar manner to that of the example of FIGs. 35-37. The frame 11 is initially formed in a square configuration (FIG. 39) with intermediately placed coiled eyelets 132 at the midpoint of each side 13, dividing the side into a first and second side portion 137,138. As depicted in FIG. 38, the temporary constraining suture 121 is used to draw the eyelets inward where they form the four additional bends 133,134,135,136 such that four valve legs 127, 128, 130, 131 are formed with the first and second side portions 137, 138 becoming sides 13 of adjacent valve legs 127, 128. A square-shaped valve orifice 117 is created when the four leaflets 78,79,119,120 are attached to the legs 127,128,130,131 of frame 11. One should appreciate that valves with more than four legs would be made in a similar manner to the examples above with a five-sided valve being formed from a pentagon, a six-sided valve being formed from a hexagon, etc.

Delivery of the device 10 can be accomplished in a variety of ways. One method, depicted in FIG. 33, involves the use of a delivery system 103 similar to that used to deliver embolization coils. The delivery system 103 comprises an outer member 105, such as a cannula or catheter, and an coaxial inner member 105 that includes a tethering tip 107, such as a notched cannula, adapted to receive a barb 17 extending from the frame 11. The tip 104 of the barb is configured such that it can positively engage with the tethering tip 107. This can be accomplished by adding a projection, such as a secondary barb, hook, spine, etc. to the tip 104, or otherwise enlarging the diameter thereof such that it can be releasably secured by the tethering tip 107 until deployment. The coaxial inner member 106 also includes an outer sheath 149 that retains and the locks the barb tip 104 within the tethering tip 107 until it is advanced or retracted by manipulation of a proximal handle (not shown) to expose the notch 150 in the tethering tip, which releases the barb 17 and deploys the device 10. The device 10 is preloaded within the outer member 105. The coaxial inner member 106 and attached device 10 are then advanced together from the outer member 106 at the target site. Further manipulation of the proximal handle, advances the tethering tip 107, which in this particular example, includes a coiled spring 151, relative to the outer sheath 149. After the device 10 has been released from the tethering tip 107, the spring-activated handle is released and the outer sheath 149 slides back over the tethering tip 107. The coaxial inner member 106 is withdrawn into the outer member 105 and the entire delivery system 103 is removed from the patient. As shown in FIG. 33, the barb tip 104 extends just beyond the coil turn 14 of the frame 11 so as to have sufficient room to engage with the coaxial inner member 106. The barb tip 104 must be positioned to account for whether the device 10 is to be placed using a femoral approach or a superior approach.

The illustrative delivery system 103 represents only one of many possibilities. For example, the device 10 can be attached to a delivery device using screws, clips, magnets, or some other tethering mechanism, or can be deployed by applying electrical current, heat, or some other means to cause detachment with a carrying mechanism. As previously disclosed, rather than making the device 10 self-expanding, where it is pushed from some sort of tubular device, it can be formed from a ductile material, mounted over a balloon or other inflatable or expandable delivery mechanism, and deployed by expanding the device in that manner.

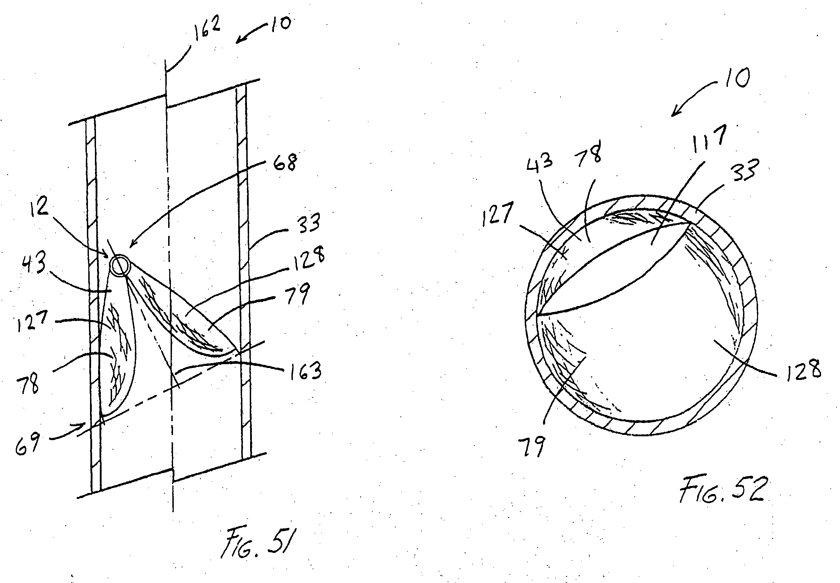

The illustrative valve examples having a simple four-point serpentine frame, such as the one depicted in FIG. 21, advantageously limit the amount of metal being placed into the vessel. This is thought to help reduce the risk of thrombus formation, as well as allow the device to assume a smaller profile in the delivery system. Because this configuration lacks some of the longitudinal stability of a frame having additional points of contact with the vessel wall (e.g., at least 4-6 at each end), there is a greater risk of the valve being deployed off-center, such as depicted in FIG. 51 and 52, wherein the longitudinal axis of the valve does not coincide with the longitudinal axis of the vessel. This, of course, results in the opening 117 of the valve being located off-center with respect to the vessel 33, which may result in the function of the leaflets 78,79 being impaired such that the valve will not properly open and close in response to blood flow.

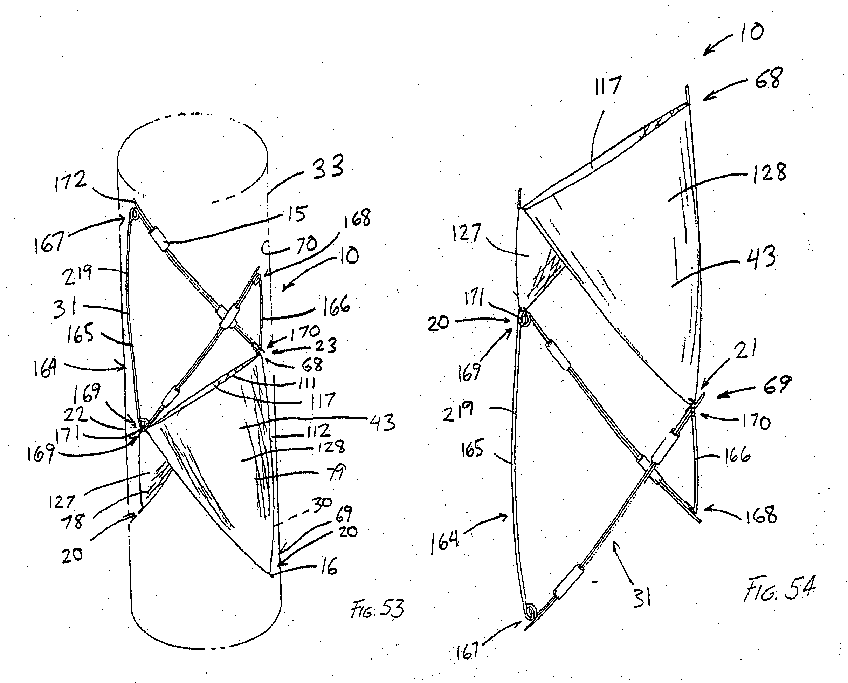

One method of addressing the problem is in the design of the delivery system, while another involves modification of the valve itself. FIGs. 53-71 depict various examples in which the valve 43 includes a centering support element 164 configured to contact the vessel wall in a manner to support and help properly align with the vessel, the basic valve portion 43 which in these particular examples, comprises a generally saddle-shaped, serpentine configuration that includes a pair of coaptable leaflets 78,79 that define an opening between two bends 20,21 comprising the first end 68 of the valve 43, the outer edges 112 of the leaflets comprising a frame 11 or resilient portion that allows the valve portion to form a seal around the entire circumference of the vessel, such that the leaflet material, preferably an ECM such as SIS, is in direct contact with the vessel wall. The centering support structure 164 is defined as a single element or plurality of elements attached to, or integral with, the valve portion 43 and which include one or more contact points 167 that engage the vessel walls such that upon deployment, the valve portion 43 is largely prevented from tilting relative to the longitudinal axis 162 of the vessel, as depicted in FIGs. 50-51, so that the opening 117 of the valve is generally centered thereinside.

The centering support structure 164 falls into two general categories. Devices 10 of the first group include support structure 164, such as second and/or third frames 31,32, an adjoining stent 219 or other expandable or inflatable elements, that are connected to, or integral with, the basic valve portion 43, and that are either attached to the proximal end, distal end, or both ends thereof. Such supporting structure 164 functions by either expanding or deploying within the vessel in advance of the valve portion such that the valve portion is less likely to tilt off-center when it fully expands, or by trailing or following the valve portion 43 out of the delivery system so that as the valve portion deploys, the centering support structure 164 remains within or attached to the delivery system to help longitudinally align the valve portion within the vessel until the support structure 164 deploys as well, as well as to prevent the valve from 'jumping' from the delivery system.

An example of a device 10 having the first type of centering support structure 164 is depicted in FIG. 53, in which the centering support is provided by a second frame 31, of the same type of the valve portion 43, attached to the valve portion by an attachment mechanism 171 such as sutures, where points 169 and 170 of the second frame 31 to bends 22 and 23, respectively. When the device 10 is deployed from the delivery system such that the second frame 31 is allowed to expand within vessel 33, the two arms 165,166 comprising the second frame expands to contact the vessel walls 70 at points 167 and 168 located at the proximal ends of respective arms 165,166 and the first or proximal end 68 of the device 10, the lateral arms basically providing a structure mirror of the two legs 127,128 of the valve. In the illustrative example, the centering support structure 164 includes a pair of barbs 172 extending from each of the arms 165,166 to further secure the device and prevent migration.

Following deployment of the leading second frame 31, the valve portion 43 is still within or secured by the delivery system, helping to maintain the second frame 31 in a longitudinally stable position. When the valve portion is deployed 43, the second frame 31, already secured in the vessel 33, provides an anchor to prevent the valve portion 43 from tilting off center as the latter expands and lodges within the vessel.

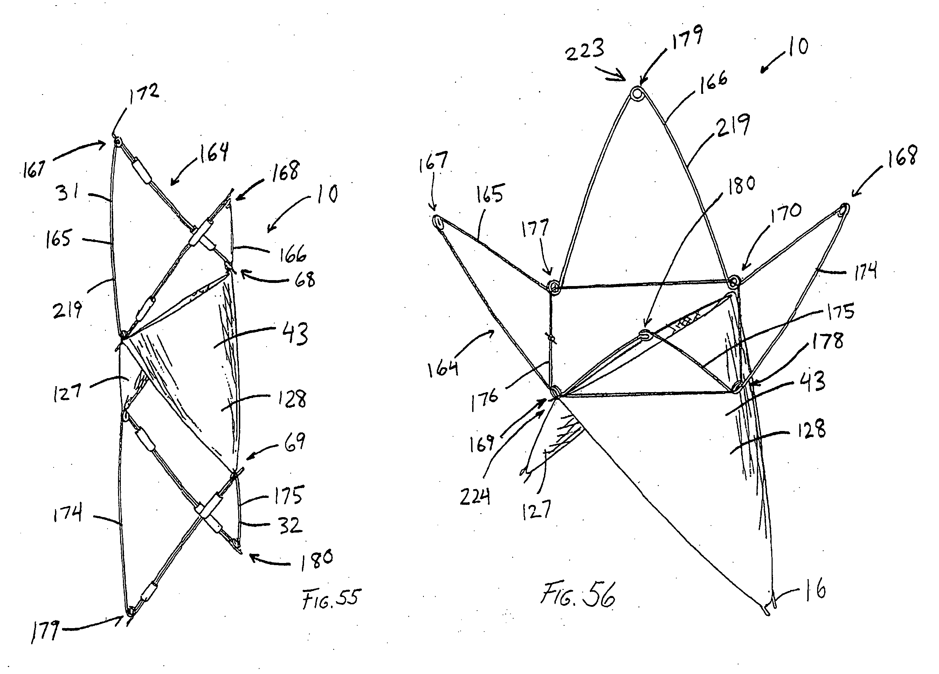

FIG. 54 depicts an example in which the valve portion 43 and second frame 31 are reversed with points 170 and 171 of the second frame being attached to bends 20 and 21, respectively, at the second or distal end of the valve portion. As the valve portion 43 is deployed, the second frame 31 remains within or affixed to the delivery system, thus reducing the likelihood of the valve portion 43 being deployed off-center with respect to the longitudinal axis of the vessel. Once the second frame 31, the remainder of the device 10, is deployed, the valve portion 43 is already positioned in the vessel, being anchored and maintained longitudinally stable by the second frame until it too is deployed, wherein the valve portion 43 in turn, provides anchoring support of the second frame to prevent it from tilting. FIG. 55 depicts an example that includes both a second frame 31 attached to the first end 68 of the valve portion 43 and a third frame 32 attached to the second end 69 thereof. Thus, the device 10 receives the centering and stabilization benefits of both centering support structure 164 components.

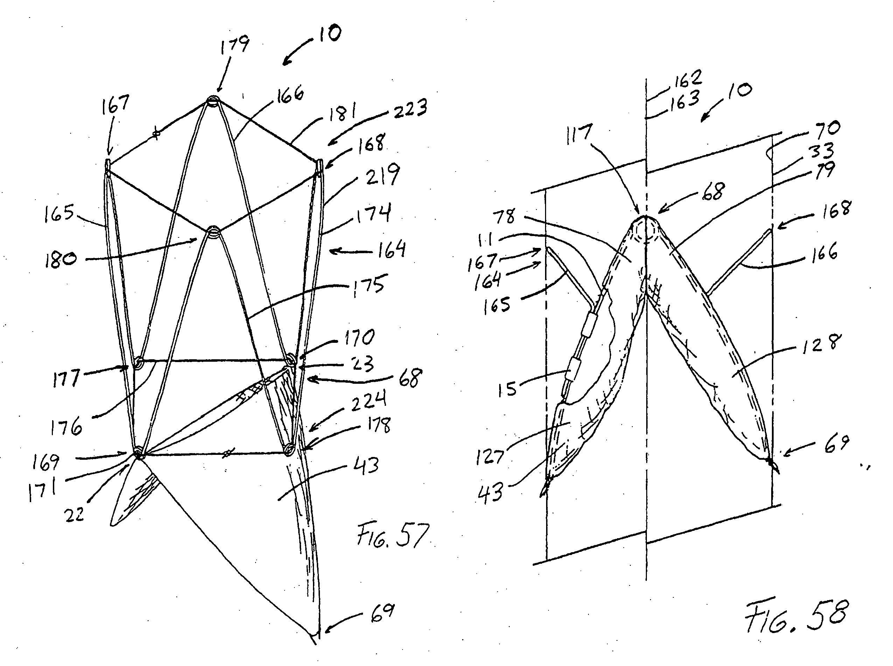

Besides having the centering support structure 164 comprise second and third frames 31,32 of the same basic four-point serpentine design, FIGs. 56-57,66-67, and 71-75 depict alternative structure that can be affixed to, or extend from, one or both ends 68,69 of the valve portion 43. FIGs. 56-57 depict an adjoining serpentine or zigzag stent 219, such as a Gianturco Z-STENT™ (Cook Incorporated) that is attached with suture 171 to the bends 22,23 located at the first end 68 of the stent. As with each of the examples, alternative attachment 171. methods can be used, such as ring fasteners, plastic bands, struts, etc. The illustrative adjoining stents 219 include a first radial constraint 176 (which is optional), such as the illustrative suture, at the second end 224 of the adjoining stent 219 to constrain contact points 177 and 178 by threading the suture 176 therethrough, along with the attachment points 169,170 of the adjoining stent 219 which are connected to bends 22 and 23 of the valve portion 43, then drawing the points inward. In the example of FIG. 57, and second radial constraint 181 is included at the first end 223 of the adjoining stent 219 which constrains the four contact points 167,168,179,180 located at that end. The illustrative zigzag stent 219 includes four points at each end; however, any number of points or configuration that allows attachment to the valve portion 43 may be used.

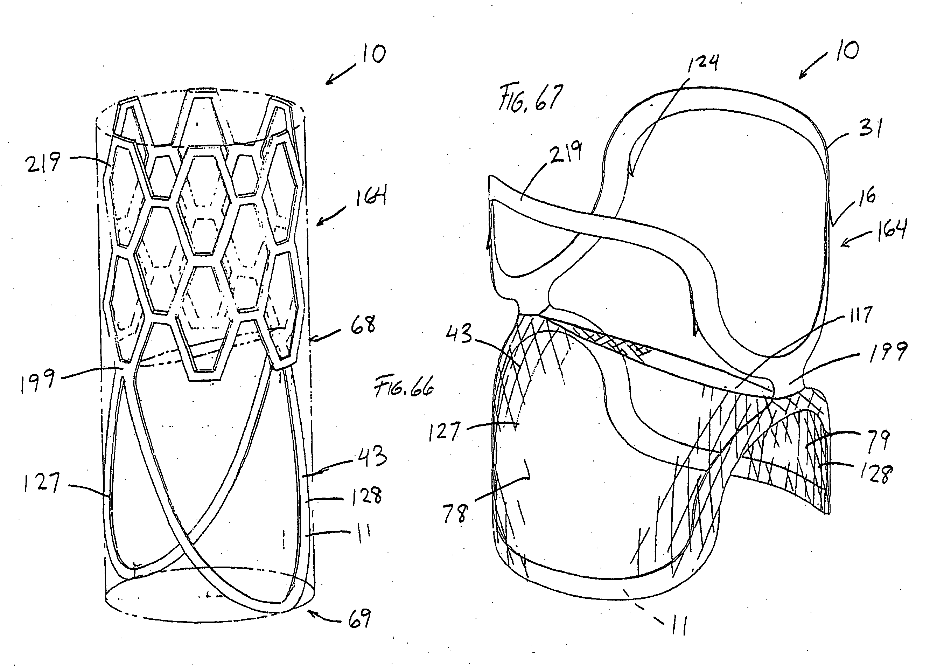

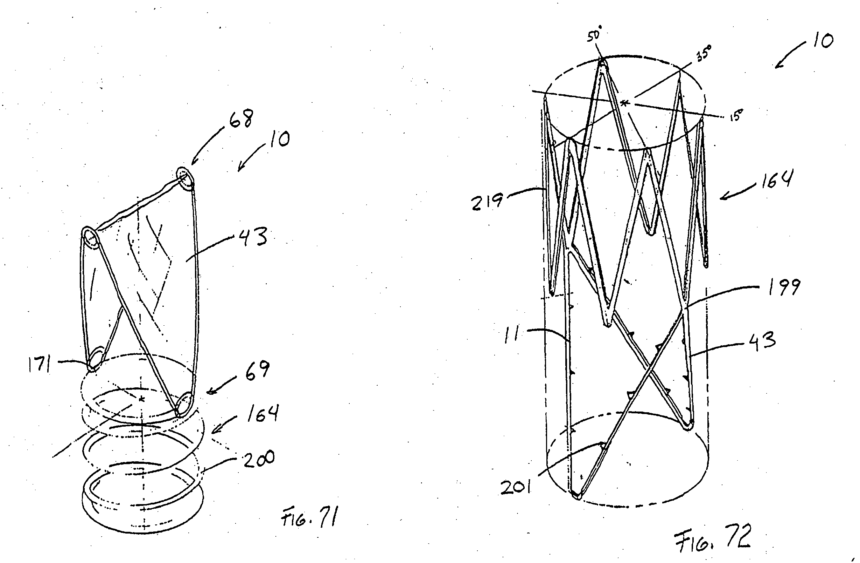

Another related example is depicted in FIG. 66 in which the adjoining stent 219 comprising the centering support structure 164 is a cannula stent such as the illustrative PALMAZ^{®} Balloon-expandable Stent (Cordis Corp., Miami Lakes, FL) that is integrally attached to the first end 68 of the stent portion 43 via a short strut 199. Both the frame 11 of the valve portion 43 and the centering support structure 164 are cut or formed from a single piece of cannula using any well-know method of forming a pattern into a cannula (e.g., laser). FIG. 67 depicts an illustrative example wherein the centering support structure 164 basically mirrors that of the stent portion frame 11, with the attachment 199 therebetween also being a short strut. FIG. 72 depicts still another example in which the both the stent portion frame 11 and the centering support structure 164 are formed from the same piece of cannula; however, the support structure 164 comprises an adjoining zigzag stent 219. An optional feature of the example of FIG. 72 are covering attachment tabs or barbs 201 distributed along the frame 11 of the stent portion, which each comprise an integral sharp projection extending from the frame 11 to help secure the covering or leaflets (not depicted) there. These tabs 201 represent an alternative or additional means of fixation to sutures.

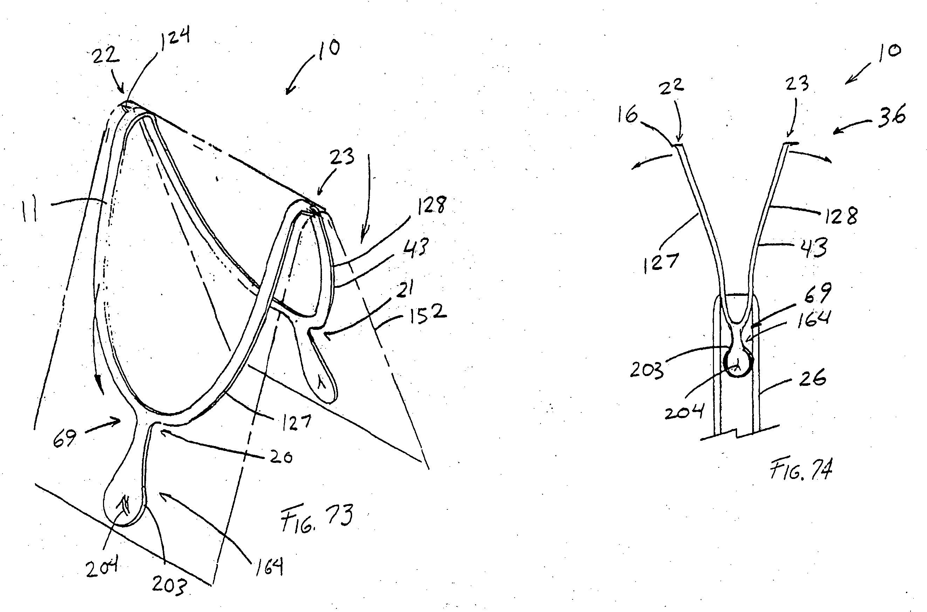

FIG. 71 depicts yet another alternative example of centering support structure 164 comprising an expandable helical structure 200 or spring that extends from the second end 69 of the valve portion 43. The helical structure 200 functions much like the aforementioned second frame or adjoining stent to remain coupled with the delivery system to help center the valve portion 43 within the vessel and/or prevent jumping of the valve. A further example of centering support structure 164 extending from the second end 69 of the valve portion is depicted in FIGs. 73-74 wherein the valve portion 43, which is cut, etched, or otherwise formed from a sheet of metals stock 152 (FIG. 73). When the frame 11 is formed into the second configuration 36, as shown in FIG. 74, the distal projections 203 are brought into close proximity to one another and become the last portion of the device 10 to be deployed from the delivery catheter 26. This allows the valve portion 43 to expand upon deployment, while still tethered to the delivery catheter or sheath 26 (FIG. 74), until the distal projections 203 exits the passageway of the delivery catheter 26, thus helping to maintain the longitudinal alignment of the valve portion 43 as it engages the vessel wall. In the illustrative example, a pair of integral barbs 124 extend from bends 22 and 23 for anchoring the valve portion 43, while the elongate projection 203 also includes an optional barb 204 for both securing the device within the vessel and providing resistance against the inner wall of the delivery system 26 to control the tendency of the device 10 to prematurely deploy when the majority of the valve portion 43 has exited the passageway. It should be noted that where the centering support structure 164 of the exemplary examples is shown extending from a particular end or another of the valve portion 43, in most instances, the support structure 164 could be easily adapted to extend from the opposite end as well.

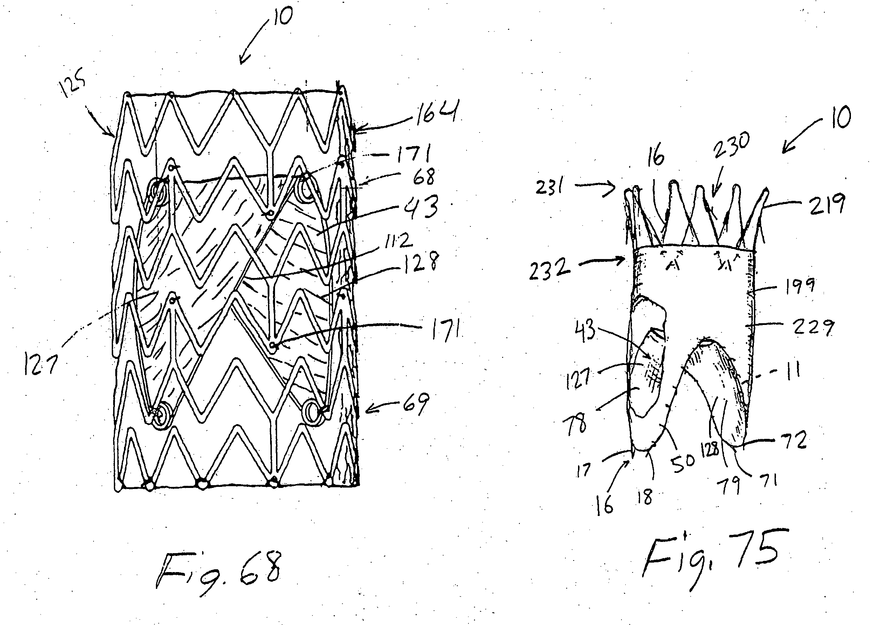

FIG. 75 depicts an example that includes both an adjoining stent 219 to provide centering support, such as the illustrative zigzag stent, as well as an outer proximal sleeve 229 that extends proximally (or upward) from the valve portion 43 and provides the attachment means 199 to the adjoining stent 219, which in the illustrative example is sewn to the first end 231 of the proximal sleeve 229 such that the stent and valve portion frames 11 do not have metal to metal contact. The second end 232 of the sleeve 229 is then attached to the valve potion 43 using sutures 50 or another well-known means. The proximal sleeve 229, which in the illustrative example has the possible advantage of not covering the vessel wall in the region between the two legs 127,128 of the valve portion 43, could optionally comprise a complete cylindrical sleeve, such as the example of FIG. 49. Optionally, the adjoining stent 219, or a second adjoining stent, could be attached to the second end 232 of the sleeve 219 if so configured. The proximal outer sleeve is preferably made of a low or non-thrombogenic biomaterial, such as SIS or another ECM. The number, configuration, and arrangement of anchoring barbs 16 can vary according to use and device configuration. In the illustrative example, a series of barbs 230 are attached to the struts of the adjoining zigzag stent 219, with the valve portion 43 including another series of barbs 17,18,71,72, as well.

A second strategy for providing better longitudinal centering support for the valve portion 43 involves the placement of the centering support structure 164, such that it extends laterally from the valve portion frame 11, rather than extending from one or both ends thereof. FIG. 58 depicts a side view of a device 10 that includes a pair of lateral arms or wings 165,166, comprising struts attached to the frame 11 of the valve portion 43. The arms 165,166, typically similar in configuration to the legs 127,128 of the valve 43, extend laterally, following deployment, to provide two supplemental contact points 167,168 that help provide longitudinal support and reduce the likelihood that the valve portion 43 would tilt off center longitudinally during or following deployment. The lateral arms 165,166 advantageously lie between the leaflets 78,79 and the adjacent vessel wall, thereby offering protection from the leaflets possibly adhering to the vessel wall 70, which could lead to failure of the valve to close properly during retrograde flow. This problem can especially occur when the valve is not properly sized (e.g., oversizing) with respect to the vessel.

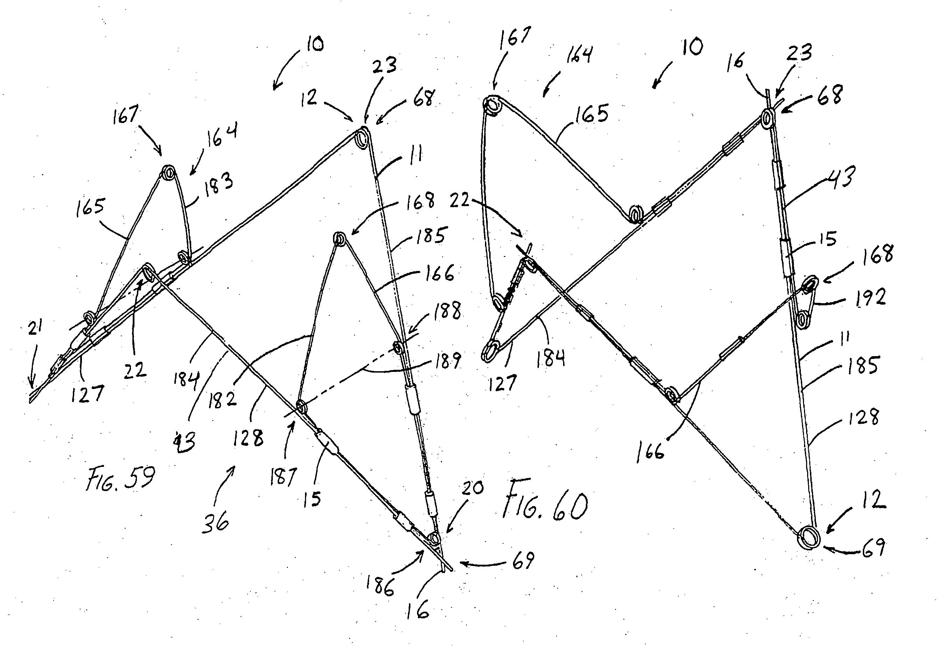

The basic example of FIG. 58 can be formed in a number of different ways, with selected examples depicted in FIGs. 59-61. The frame 11 of the example of FIG. 59 comprises four components. The lateral arms 165,166 each comprise part of a closed diamond-shape component 182,183. For example, lateral arm 166 includes four bends 168,186,187,188, with bend 168 comprising the contact point of the lateral arm 166 and bend 186 forming the bend 20 of the valve leg 128. To permit the arm 166 to extend outward from the valve portion 43 (shown without leaflets) so that it is able to help in centering, bends 187 and 188 deformed in a different plane, such that the closed frame 182 is bent along an axis 189 intersecting both bends. The angle of the bend should be such that the contact point 167,168 exerts a safe, but effective pressure against the vessel wall when the valve is in the deployed configuration 36, such that the valve 43 is unlikely to tilt. Each closed section 182,183 is attached to a pair of V-shaped sections 184,185 which each include a bend 22,23 that together, comprise, the first end 68 of the valve portion 43. It should be noted that the term 'V-shaped' also includes the concept of a rounded 'V' or 'U-shaped' section as well. The components 182,183,184,185 can be joined by soldered cannulae, laser or spot welding, or some other well-known means of joining a metal frame. Once assembled into a closed frame 11 having lateral arms 165,166 comprising the centering support structure 164, the ends of the V-shaped sections can serve as anchoring barbs 16 to secure the valve 43 following deployment.

FIG. 60 depicts an alternative assembly of the basic example of FIG. 58. Like the example of FIG. 59, the device 10 includes two V-shaped section 184,185, which in the illustrative example comprise the two legs 127,128 of the valve portion 43. The remaining component comprises a serpentine portion 192 which comprises an eight-point zigzag stent in which two of the points 167,168 (those oriented toward the first end 68), and their adjacent struts, form the arms 165,166 comprising the centering support structure 164. The other two points of the serpentine portion 192, adjacent to points 167 and 168, comprise bends 22 and 23 of the valve portion 43, when the components are assembled. The ends of the V-shaped sections 184,185 form barbs 16 at the first end 68 of the device. Optionally, separate barbs can be attached to the frame 11, such as using the illustrative cannulae 15, if barbs are desired at the second end 69.

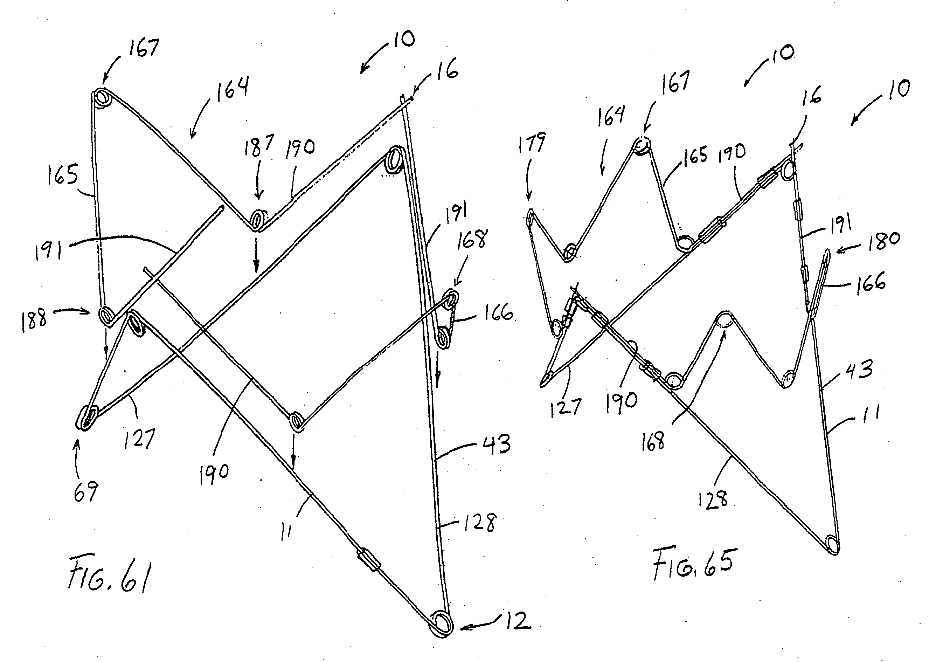

A third example, similar to those of FIGs. 59 and 60, is depicted in FIG. 61. In this example, the valve portion 43 is basically the same serpentine frame 11 configuration as most of the illustrative valve examples (excluding those of FIGs. 59 and 60). The arms 165,166 comprising the centering support structure 164 each include a pair of attachment struts 190,191 that lie parallel to the legs 127,128 and are attached to the frame 11 thereof, using cannulae (now shown), welding, or another well-known method. The open ends of the attachment struts 190,191 serve as barbs 16 extending from the first end 68 in the illustrative embodiment. It should be noted that the components of the embodiments of FIGs. 58-61 can either be made of the same material and strut thickness, or they can be formed of different materials. For example, in the example of FIG. 61, the valve portion 43 frame 11 might be made of spring stainless steel, while the arms 165,166 are made of nitinol or a smaller gauge of stainless steel wire. Additionally, the arms 165,166 might be made of bioresorbable material or biomaterial which would help center the valve portion, then be resorbed or disappear after the valve has stabilized and thus, be unlikely to tilt, or if adherence of the leaflets to the vessel is not a concern.

FIG. 65 depicts an example similar to that of FIG. 61 except that the arms 165,166 are configured such that they include both a first contact point 166,167 and a second contact point 179,189 that each contacts the vessel wall 70 for further stability. The M-shaped arms 165,166 (the portion extending outward from the valve portion 43) further include a pair of attachment struts 190,191 that are affixed to the valve portion frame 11 and may extend outward to form anchoring barbs 16.

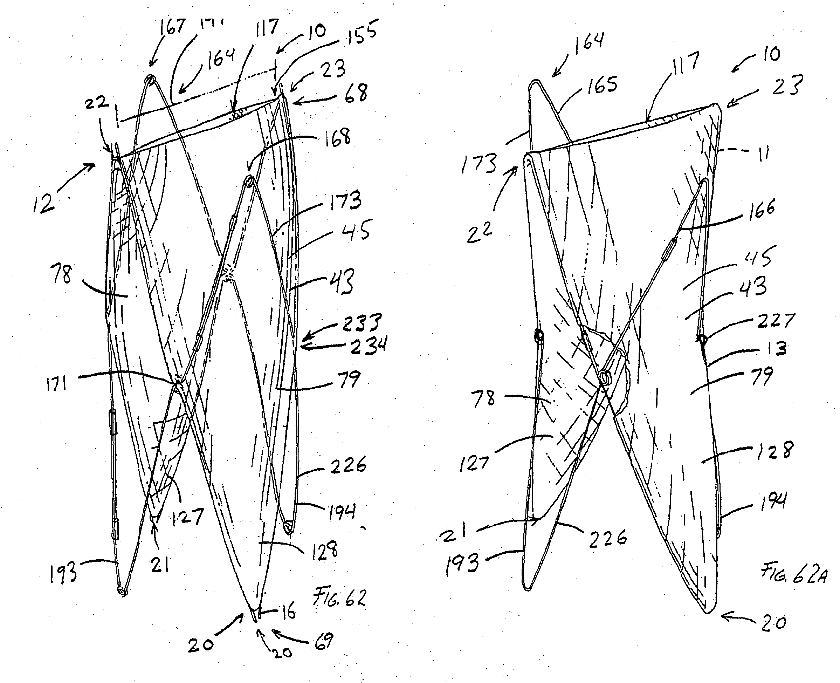

FIGs. 62-62A depict similar examples in which the centering support structure 164 includes both a set of lateral arms 165,166 as in the previous examples, along with a pair of supplemental legs 193,194 for additional longitudinal support. In the example of FIG. 62, the device 10 comprises a first and a second serpentine elements or zigzag stent portions 174,226 attached end to end using an attachment mechanism 199 such as suture. The legs 127,128 then span both zigzag portions 174,226, such that bends 22 and 23, located at the first end of the valve portion 43, are part of the first zigzag portion 174, while bends 20 and 21, located at the second end 69 of the valve portion 43, are part of the second zigzag portion 226. The example of FIG. 62A comprises a single frame 11 which also forms the lateral arms 165,166 and the supplemental legs 193,194, as well as the legs 127,128 of the valve portion 43. The legs include a loop 227 located midway on the sides 13 that provide an attachment point to the crossing struts of lateral arms 165,166 supplemental legs 193,194, which optionally include attachment loops 227.

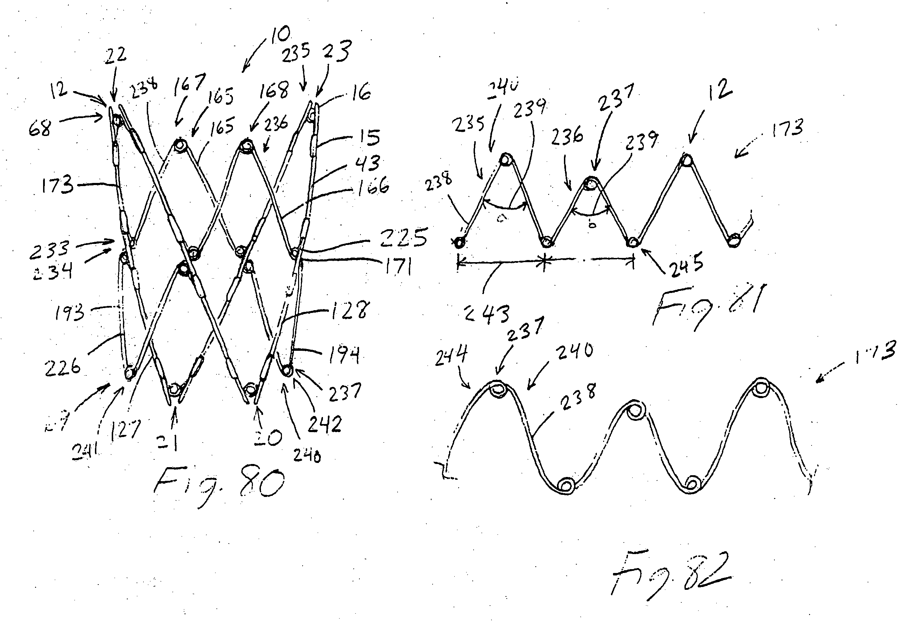

The examples of FIGS. 62-62A are but two examples of a method of forming a valve portion 43 having two lateral arms 165,166 and two supplemental legs 193,194. FIG. 80 depicts a frame 11 of yet another example whereby the first and second serpentine elements 173,226 are joined by a series of elongate valve leg struts 225 that are attached to selected struts 238 of the serpentine elements 173,226 with cannulae 15 or another well-known method of bonding such that the individual serpentine sections 240 across the two serpentine elements 173,226 form the legs 127,128 of the valve 43 to which the leaflets 78,79 are attached (similar to that depicted in FIG. 62). For purposes of the present disclosure, a serpentine section 240 is defined as a bend 237 and the accompanying struts 238 that originate therefrom to assume a V-shape component, which connects to adjacent bends 245 oriented in the opposite direction to form the 'zigzag' or 'Z' or 'S' configuration. In addition to comprising the attachment mechanism 171 that joins the two serpentine elements 173,226, the leg struts also provide added rigidity to the legs 127,128 and frame 11 so that the weight of column of blood acting on the leaflets is less likely to cause the top bends 22,23 to be pulled inward toward one another, thereby changing the shape of the valve 43, which could affect the coaptation of the leaflets (how well they fit together or contact one another) and perhaps, lessen the radial pressure or force being applied against the vessel wall. Furthermore, the leg struts 225 may be conveniently extended beyond each of the bends 20,21,22,23 such that they form a series of eight barbs (two at each bend with four oriented in the proximal direction and four in the distal direction, to anchor the device 10 following deployment.

Unlike examples of the present invention in which the frame 11 can be flattened to facilitate attachment of the covering or leaflets, the covering must be attached to the tubular-shaped illustrative examples of FIGs. 62-62A and 80, and others, by another means. One exemplary method includes introducing a wedge or chisel-shaped mandrel into the lumen of the device over which a wet, diamond-shaped piece SIS or other covering 45 is placed such that it conforms and assumes its characteristic saddle-shaped configuration. The covering is closely aligned with the frame 11 of the valve portion, then the covering 45 is attached along the axis that includes the orifice 117 by hooking the barbs 16 located at bends 22 and 23 therethrough. The covering 45 is then attached in a similar manner using the barbs 16 at ends 20 and 21. The mandrel diameter is controlled to result in a deployed valve that produces the desired amount of coaptation of the leaflets and other performance characteristics. The covering is then sutured in the manner similar to that depicted in FIGs. 26-26A. To attach the covering around that point which the lateral arms 165,166 and supplemental legs 193,194 extend from the frame 11, a slit is made a corresponding point along the covering that allows the struts to emerge, then the covering is wrapped therearound and secured in place. After the covering has dried, it is slit along the short axis to form the orifice, preferably leaving a 1-2 mm gap of covering remaining between the bend 12 and the edge of the orifice 117 to help prevent reflux at the corner bends 22,23. The mandrel may be removed before or after the drying process.

Referring now to FIGs. 80 and 81, the lateral arms 165,166 and supplemental legs 193,194 that comprise the centering support structure 164 in the illustrative example are made up of serpentine sections 440 comprising alternating lateral arm serpentine sections 236 and valve leg serpentine sections 235, the latter comprising a portion of the frame 11 supporting the two legs 127,128. In the illustrative example, the valve leg serpentine sections 235 are longer than the lateral arms section 236 (and lateral arms 165,166). For example, the struts 238 of the lateral arm serpentine sections 236 may be 12 mm, as opposed to 15 mm for the struts 238 of the longer serpentine sections 235. Shortening the lateral arms 165,166 and supplemental legs 193,194 relative to the adjacent serpentine sections 235, helps keep the ends 167,168,241,242 thereof away from the bends 20,21,22,23 and barbs 16 of the valve portion 43 for easier loading and less chance of entanglement during deployment. Conversely, the lateral arms 165,166 and/or supplement legs 193,194 can be made longer (e.g., 20%) to also avoid having the ends becoming ensnared with barbs. A further advantage of a longer lateral arm 165,166 is that the leaflets 78,79, as they open and are folded back during valve function, cannot become caught on the contact points 167,168, which in the case of the longer arms, is well above the reach of the leaflets.

In addition to varying the length of the serpentine sections 235,236 to change the performance characteristics of the valve 43 (with leaflets not shown), the width 243 of the respective sections 235,236 can be varied, as well as the angle 239 formed by the bend 237 bend 12 (eyelet) diameters, and struts 238, to create a valve that exerts the desired radial pressure against the vessel such that it properly seals with the vessel without causing erosion of vessel wall tissue due to excess force. These dimensions can be changed to maintain a constant radial pressure across the range of different valve sizes. Furthermore, these dimensions can be manipulated to produce other desired characteristics, such as minimizing the amount of plastic deformation the frame 11 undergoes upon being loaded into the delivery system. In the illustrative artificial venous valve example, the preferred range of the frame 11 wire thickness is 0.003-0.030", with a more preferred range of 0.0075-0.015" and most preferred range of 0.008-.012". The preferred eyelet diameter at the bends 15,237 is 0.005-0.150", with a more preferred range of 0.010-0.060" and a most preferred range of 0.015-0.040". The length of the strut 238 of the valve leg serpentine section 235 is preferably 3-25 mm, with a more preferred range of 7-16 mm. The lateral arm serpentine section 236 is preferably 3-30 mm, with a more preferred range of 5-19 mm. Preferably, the struts 238 of the lateral arm serpentine section 236 should be about 80% of the valve leg serpentine section 235 or 20% longer, to give the desired amount of offset to avoid entanglement. Referring also to FIG. 62, the overall length of the illustrative device 10 (combined serpentine stents 173 and 226) should preferably be 1-2X the vein diameter, with a more preferred range of 1.5-2X. The valve covering 45 is sized so that the orifice 117 is able to open to 10-120% of the vein diameter, with a more preferred range being 60-100%. The preferred range of the amount of coaptation or contact of the leaflets 78,79 is 5-150% of the diameter of vein where the valve is implanted, with a more preferred range of 10-50%

FIG. 82 depicts a flattened portion of a first or second serpentine stent 173 in which the struts 238 are plastically deformed into a curved configuration 244 such that the serpentine sections 240 are more rounded and are able to better conform to the vessel wall. To maximize or preserve the curvature of the struts 238, the cannulae 15 attaching the valve strut 238 (as depicted in FIG. 80) can be located more toward the center of the respective struts 238 being joined. Otherwise, only the portions of the struts 238 about the bends 237 would assume the rounded or curved configuration 244 in the assembled device 10.

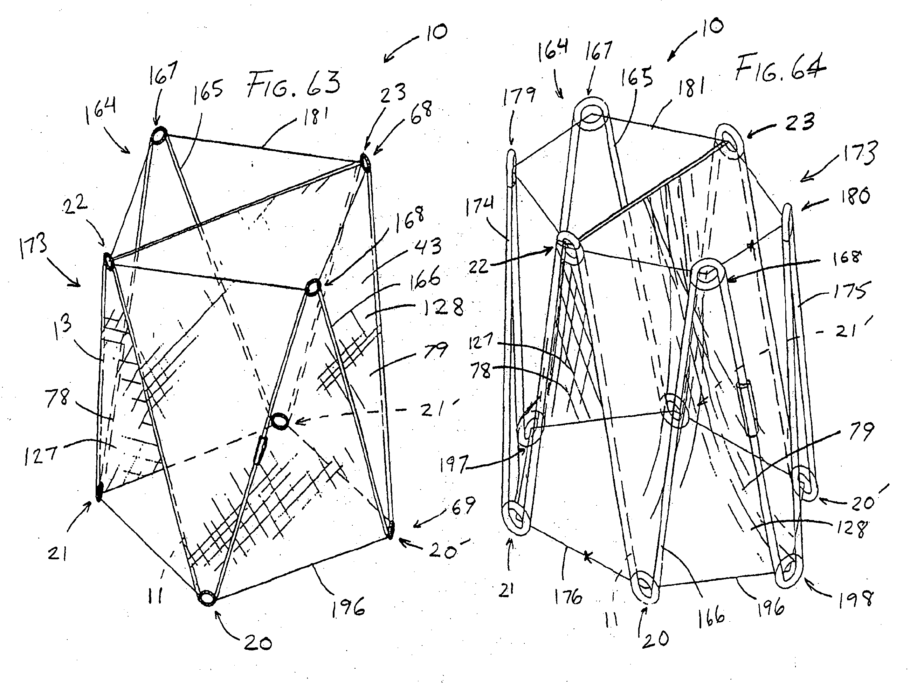

FIGs. 63-64 depict valve examples in which frame 11 and centering support structure 164 are part of common structure comprising a first serpentine or zigzag stent 173. In the example of FIG. 63, the eight-point zigzag frame includes four distal 20,20',21,21' and four proximal 22,23,165,166 bends. The struts or sides 13 that connect points 22 and 23 provide the frame 11 support of the leaflets 78,79. Rather than supporting the entirety of each leaflet 78,79, the distal or bottom edges 196 thereof are not reinforced by the frame 11. An optional supplemental frame (not shown) may be included to reinforce the distal edges 196 and give it added resiliency for sealing against the vessel wall. FIG. 64 depicts a similar example to that of FIG. 63 in which the frame 11 and centering support structure 164 are a twelve-point zigzag stent, such that there are both first 165,166 and second 174,175 pairs of arms. The distal or bottom edge 196 of each leaflet 78,79 is attached to a distal contact point 198. As with the example of FIG. 63, the distal edge 196 can be made resilient by the addition of a wire or other materials that help it provide a better seal against the vessel wall.

FIG. 68 depicts an valve example that is related to that of FIG. 49 in which the valve portion 43 includes a circumferentially constraining mechanism 125. Unlike the sleeve or material depicted in FIG. 49, the circumferentially constraining mechanism 125 of FIG. 68 example comprises a radially expandable or self-expanding stent, such as the illustrative ZILVER™ Stent (Cook Incorporated) which is made of a superelastic NiTi alloy. In the illustrative example the circumferentially constraining mechanism 125 is particularly adapted to serve as a centering support structure 164, although even the material sleeve of FIG. 49 would offer a similar benefit, as well. The valve portion 43 can either be sized to fit within the outer stent such that it would not migrate, once deployed, or the legs 127,128 could be sutured or otherwise affixed to the legs using an attachment mechanism 171 to ensure that the valve portion 43 does not move relative to the constraining mechanism 125.

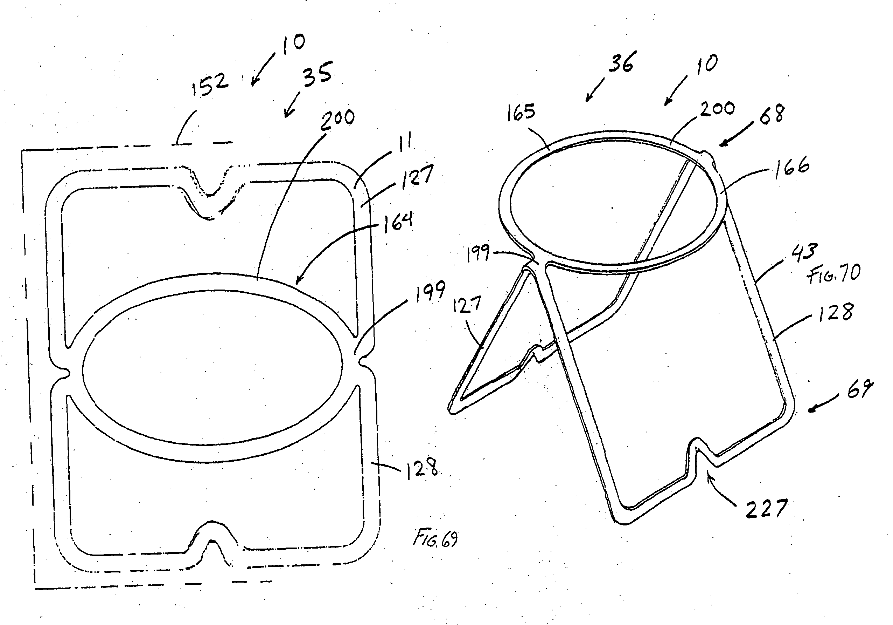

FIGs. 69-70 is formed a sheet of material 152, such as stainless steel, nitinol, etc., by laser cutting, stamping, machining, etching, or some other well-known method, wherein the design includes a valve portion 43 and integral centering support structure 164 that is folded or otherwise reshaped from the flat, first configuration 35 into the second, deployed configuration 36. In the example of FIGs. 69-70, the centering support structure 164 comprises a pair of opposing arms 165,166 that extend outward when the device 10 is in the deployment configuration 36 such that they form a circular ring configured to support the valve portion 43 and prevent it from tilting within the vessel. In the illustrative example, the centering support structure 164 is attached to the frame 11 of the valve portion 43 by a pair of short struts 199. One or more optional flexible zones 227, that comprise bends in the frame 11, may be incorporated thereinto to for the purpose of providing better conformity of the frame to the vessel.

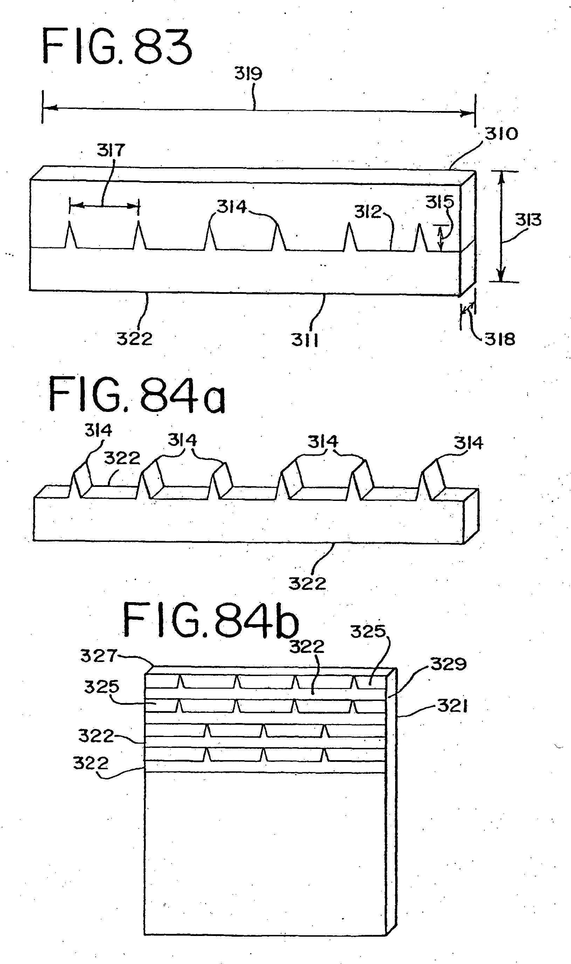

In the context of FIGs. 83-92, the term distal with respect to a prosthesis or stent graft is intended to refer to the end of the prosthesis or stent graft furthest away in the direction of blood flow from the heart and the term proximal is intended to mean the end of the prosthesis or stent graft which when implanted would be nearest to the heart.

FIG. 83 shows a flat wire 310 from which a stent of the present invention can be manufactured. The line or barb profile 312 shows a pattern with which the stent with integral barbs may be cut. The size of the wire 310 preferably relates to the desired dimensions of the final stent, including the barbs. The width 318 of the flat wire 310 should approximate the desired thickness of the final stent wire. The length 319 of the wire 310 should accommodate the desired length or circumference of the final stent wire. The height 313 of the flat wire 310 should accommodate the sum of the desired thickness of the final stent wire 322 plus the length 315 of the barbs 314.

The flat wire 310 can be made of stainless steel, nitinol, any other suitable metal or any other material known to one of skill in the art. The material can have shape memory properties or not. Any other shape wire can be used, including, for example, round wire or beveled flat wire. Any kind of flat wire, including the flat wire 310 shown in FIG. 83 is a "sheet" of stent material; any other shape of a sheet can be used. Furthermore, the term wire encompasses any generally slender body, regardless of how it is made and regardless of any surface features that may extend from it. A wire can have two ends or have its ends connected to form a circular shape.

The barb profile 312 can be varied. In particular, the distance 317 between barbs 314 and the height 315 and shape of the barbs can be varied. The opposing side 311 of the flat wire 310 may also be cut to provide additional barbs. The desired barb profile can be determined by the level of traction that the particular application requires and/or concerns about the damage to the surrounding vessel that may be caused by the barbs, *inter alia.*

FIG. 84a shows the flat wire of FIG. 83 after the barbs 314 have been cut into the stent wire 322 or stent segment. The flat wire 310 can be cut in any way known to one of skill in the art. The flat wire 310 is preferably cut using lasers, but may be stamped using metal stamping techniques, or cut by sawing, power hacksawing, shearing, abrasive cutting, plasma or thermal cutting. The laser may have a power of about 1800-2800W or any other suitable power; nitrogen or any other suitable gas may be used as an assist gas.

The starting material for manufacturing the stents of the present invention is preferably a flat wire 310 such as that shown in FIG. 83, but can also be a long, flat sheet 321, as shown in FIG. 84b. If the starting material is a long sheet 321 of metal or similar material, then the barb profile 325 can be cut into the perimeter 327 of the long sheet 321. Then the long sheet 321 can be cut at line 329 to separate the stent wire 22 and barbs. Stent wires 322 can be cut one-by-one through the entire long sheet 321 in this manner. The cutting techniques described above can be employed.

Following the cutting process, the stent wire 322 or stent segment may be rough in areas or have sharp or jagged edges, or other surface defects. Stress tends to concentrate around those surface defects. Therefore, the properties of the stent wire 322 or stent segment are generally improved when those surface defects are removed. Surface defects are preferably removed by electro-polishing but may also be removed by mechanical polishing, chemical polishing, or any other method known to one of skill in the art.

Electro-polishing is the electrolytic removal of a metal in a preferably highly ionic solution by means of electrical potential and current. Electro-polishing is preferably used to smooth, polish, de-burr or clean an electrically conductive material. It removes stress concentrations by selectively removing surface defects on metal surfaces, thereby making the material stronger. Electro-polishing can also improve corrosion resistance and remove hydrogen from the surface of the stent.

The electro-polishing process preferably begins with the preparation of the stent by cleaning it, which can remove non-conductive material from the surface of the stent. Oils, glues and other substances are possible contaminants. Then, the stent can be electro-polished by placing it in an acid bath, preferably a phosphoric and sulfuric acid solution, and connecting the positive lead of a DC power supply to the stent and a negative lead to a cathode. Post-treatment preferably involves placing the stent in a nitric acid rinse followed by a water rinse.

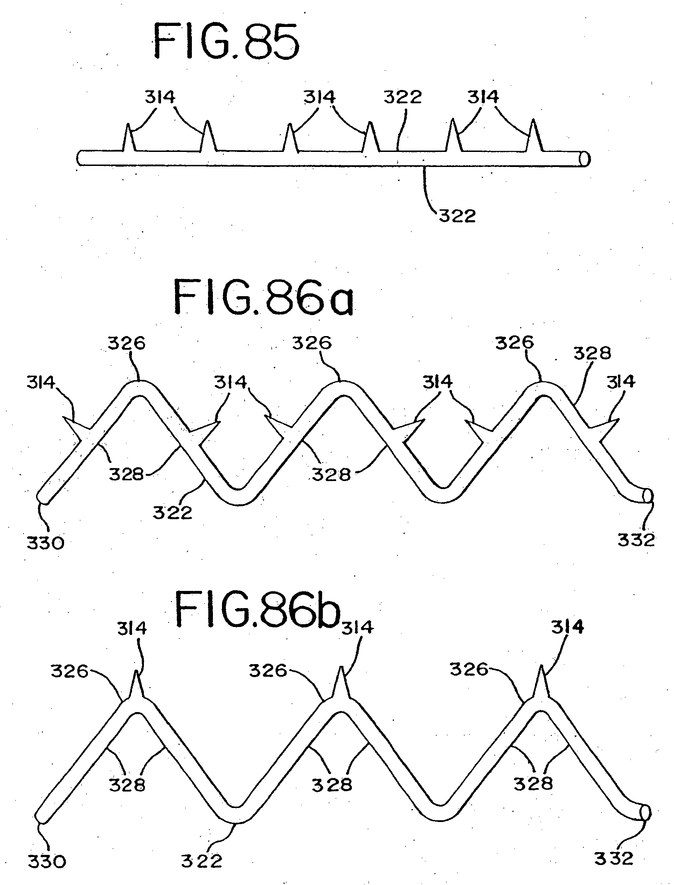

FIG. 85 shows the stent wire 322 following the polishing process. All of the edges and rough spots have been smoothed out, but the barbs 314 remain.

After the polishing process, the stent wire 322 of FIG. 85 can be bent into a suitable stent wire shape, preferably a zigzag shape. Any other shape can be used, including a sinusoidal shape. Any suitable frequency and amplitude can be employed.

Before the bending step, in accordance with the present invention, barbs 314 are properly oriented so that they will point in the desired direction in relation to the longitudinal axis of the final stent shape. This obviates the need for bending the barbs 314 relative to the stent wire 322. Such an approach to barb orientation is preferable because bending the barbs 314 can, in some cases, weaken them.

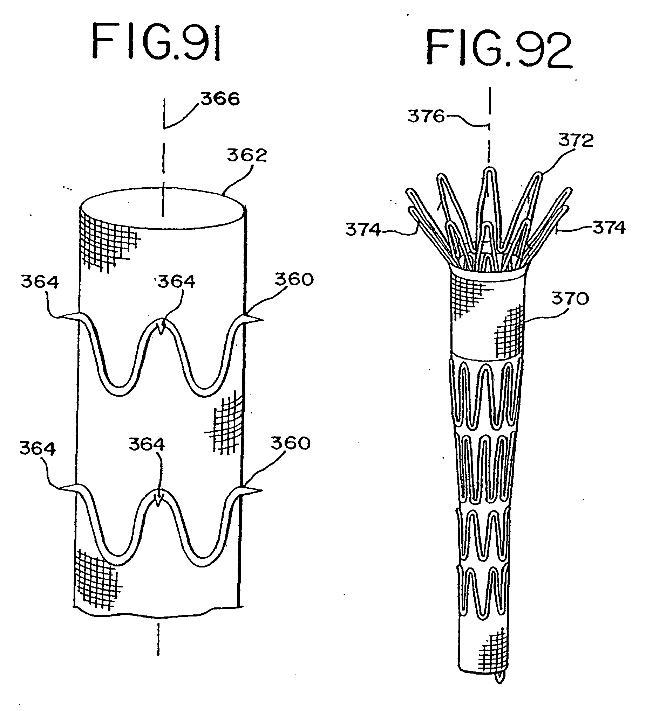

If the stent is used as a proximal fixation stent or similar type of stent, it is preferred to have the barbs 314 angled so that they are generally transverse to the longitudinal axis. It is also preferred that an acute angle is formed by the barbs and the distal longitudinal axis of the final stent shape, as described below and shown in FIG. 92. This ensures that the barbs "catch" and engage the adjacent tissue.

FIG. 86a shows the stent wire 322 of FIG. 85 bent into a zigzag shape. In the particular embodiment shown in FIG. 86a, the barbs 314 are positioned on the stent wire 322 near the midpoint of the struts 328 which extend between the zigzag peaks or bends 326. As shown in FIG. 86b, the barbs 314 can also be placed on alternating zigzag bends 326. Barbs can also be placed on all of the bends or on a combination of struts and bends.

Following the bending process, or at other steps prior to the bending process, the ends 330, 332 of the stent wire can be connected by any means known to one of skill in the art, including wrapping, chemical bonding, welding, brazing, soldering or other techniques. If the stent is shaped helically or otherwise, there may be no need for such a connection.

Many of the steps described above in reference to FIGs. 83-86 can be ordered differently. For example, it is possible to cut the wire, connect the ends, and then polish it. One could also connect the ends before bending a zigzag or other shape into the circular stent.

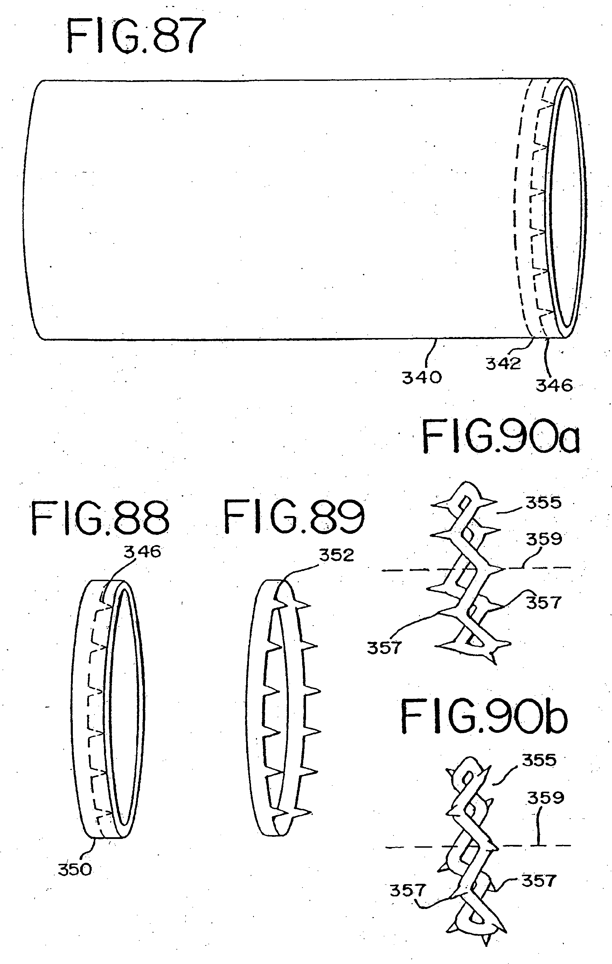

As shown in FIG. 87, the stent with integral barbs of the present invention can also be formed from a cannula 340. Formation of the stent from a cannula 340 obviates the need to connect the ends of the stent wire. FIG. 87 shows a relatively thin walled cannula 340 suitable for manufacture of the stent of the present invention. The cannula 340 can be made of stainless steel, nitinol, any other suitable metal or any other material known to one of skill in the art.

The barb profile 346 can be cut into one end the cannula 340. Then a length of the cannula 340 can be cut from the remainder of the cannula 340 at line 342. Also, as shown in FIG. 88, a section 350 of a selected width may be cut from the cannula 340; then the barb profile 346 can be cut.

FIGs. 87 and 88 show a possible barb profile 346 for the stent of the present invention. The barb profile 346 can be varied for the desired barb frequency, size and shape. These variables can be determined by the level of traction that the particular application requires or to minimize the damage to the surrounding vessel.

The cannulae of FIGs. 87 or 88 can be cut in any way known to one of skill in the art including the methods cited above. The resulting stent wire or stent ring 352 is shown in FIG. 89. Once the stent ring 352 is cut from the cannula 350, the stent ring 352 can be polished as described above. Following polishing, the stent ring 352 can be bent into the desired shape.

A final stent shape is shown in FIG. 90a. The particular stent 355 is a zigzag or Z-stent. One example of such a stent is the Gianturco Z-stent. This shape can be formed by the method described in relation to FIGs. 83-86 or the method described in relation to FIGs. 87-89. A stent resulting from the method described in relation to FIGs. 83-86 will have a seam or other junction where the ends of the stent wire are connected. The stent 355 has a longitudinal axis 359. The stent 355 has barbs 357 cut into both sides of the stent wire. As shown in FIG. 90b, the barbs 357 are preferably pointing in a generally transverse direction in relation to the longitudinal axis 359. By pointing outward from the longitudinal axis 359, the barbs 357 can engage the adjacent tissue when they are deployed. Any other stent shape can be employed, including a sinusoidal shape.

Many of the steps described above in reference to FIGs. 87-90 can be ordered differently. For example, it is possible to bend the wire before or after polishing it.

In FIG. 91, there are two zigzag stents 360 of the present invention attached to a prosthetic vessel 362 to form an endoluminal prosthesis or stent-graft. The stents 360 can be attached by any means known in the art, including by suturing. Barbs 364 are shown extending generally transversely from the longitudinal axis 366 of that prosthetic vessel 362. The angle of the barbs 364 relative to the longitudinal axis 366 can be changed by bending them.

In FIG. 92, stents 372 of the present invention are shown as a part of an aortoum-iliac prosthesis 370. The barbs 374 extend from suprarenal fixation stents 372. The barbs 374 are not directly perpendicular to the longitudinal axis 376; they are generally transverse, but point in the distal direction, thereby forming an acute angle with the longitudinal axis 376. Such a suprarenal fixation stent 372 with integral barbs 374 can also be used to secure a bifurcated aortic prosthesis or similar prostheses. The stent 372 is designed with long struts so that they extend over and past the renal ostia when the prosthesis 370 is deployed.

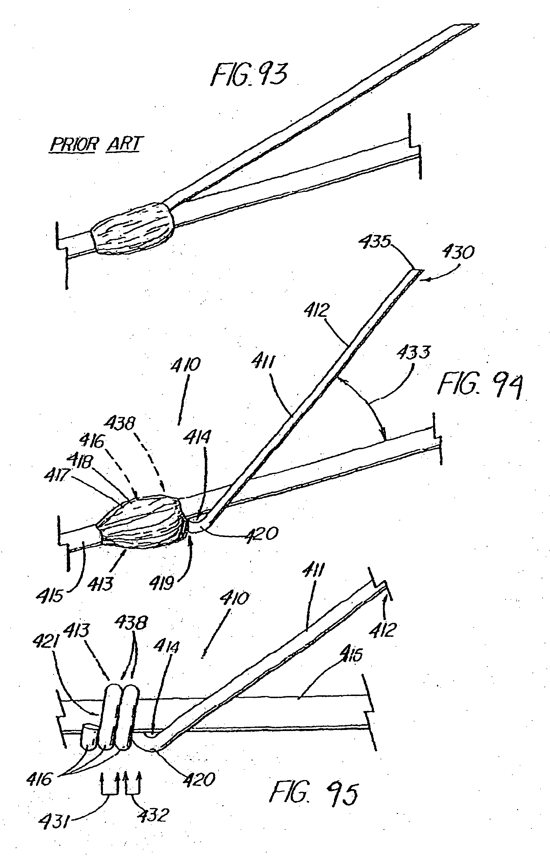

FIGs. 94-100 depict a medical prosthesis 410, such as a stent, stent graft, valve, vessel occluder, filter, or other intraluminal medical device, that includes one or more barbs 411 that comprise an anchoring portion 412 sized and oriented to engage tissue for the purpose of anchoring the device and preventing the downstream migration thereof; a basal portion 413 located about the physical union between the barb and the strut of the prosthesis 410 to which it is affixed; and a stress-dispersing region that forms a transition between the basal portion 413 and anchoring portion 412 of the barb 411. The stress-dispersing (or stress-reducing) region 414 comprises a section of the barb that has been shaped and configured to receive most of the forces acting upon the anchoring portion 412 or moment arm of the barb as it bends and distribute them throughout that region 414, rather than allowing them to be concentrated at a single point or relatively narrow region, such as the point of union 419 between the barb 411 and substrate of origin 415, the substrate of origin 415 typically being a strut 415 of a intraluminal stent or other prosthesis to which the barb 411 is attached. The term 'strut' 415, as defined herein, may encompass a wire, bar, bend, or any portion of the prosthesis from which the barb may emanate, and is not necessarily limited to a strut as traditionally defined in the medical arts, typically meaning a thin section of the metal framework of a self-expanding or balloon-expandable stent. For example, the barb may be sewn or otherwise attached directly to graft material or another portion of the prosthesis, or it may be formed integrally with the prosthesis. Additionally, the barb may be slidably affixed to the strut 415 to at least temporarily help relieve stresses about the point of union 419, which is generally defined as that point where the barb extends away from the substrate of origin 415 and/or the means of mechanical attachment 417 or bond 418 between the two.

It should be understood that the delineations between the anchoring portion 412, the stress-dispersing portion 414, and basal portion 413, while primarily functional in nature, are not absolute. The basal portion may represent a well-defined and distinct section of the barb, or merely represent the point of attachment or union with the strut 415 or framework of the prosthesis 410. In addition, the stress-dispersing region 414 may extend sufficiently away from the strut 415 that it also may penetrate adjacent tissue and serve to help anchor the stent. Generally, however, the stress-dispersing region 414 is located proximate to the point of union 419 such that the anchoring portion 412 provides most of the anchoring function. Although the addition of structure for reducing moment of stress can be placed anywhere along the length of the barb 411, it is most advantageous when located near the base thereof (point of union 419), especially if the stress load is being placed over a significant portion of the barb's length. For example, a series of bends or coils located exclusively at the midpoint of the barb 412 would provide little, if any, stress-relieving value if those bends become imbedded in tissue. In such a situation, the stress moment caused by the torsional and other bending forces acting on the barb would be transferred down toward the barb's base where stress-dispersing structure is lacking.

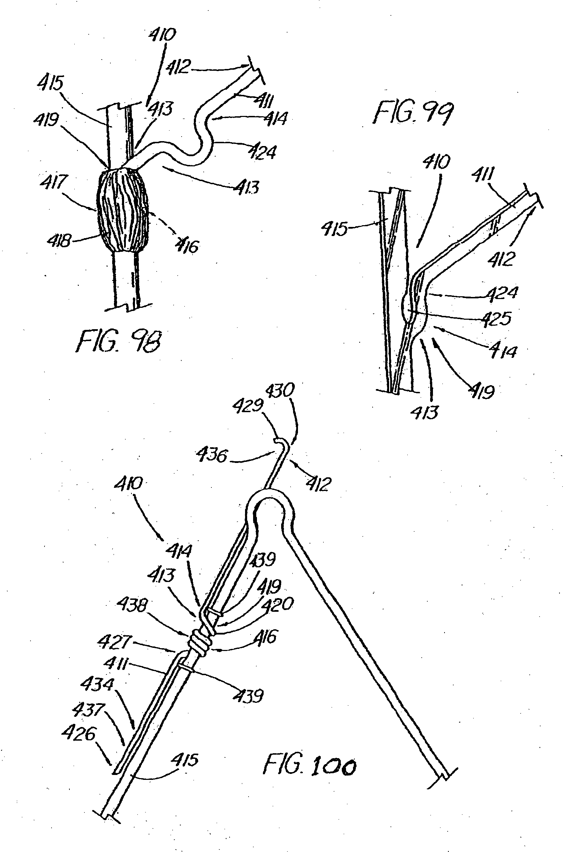

FIGs. 94 and 95 depict an illustrative example of a type of barb 411 that includes a helical coil 438 that is wound around the strut 415 to which it is attached. The barb 411 in configured to anchor a stent or other prosthesis, such as the suprarenal stent of a endovascular stent graft, such as a ZENITH™ AAA Endovascular Graft (Cook Incorporated) used to treat an abdominal aortic aneurysm (AAA) located in the vicinity of the aortic bifurcation. A series of staggered barbs are affixed to the proximal, suprarenal Z-STENT™ (Cook Inc.) to anchor the stent graft within the proximal neck of the aneurysm being treated and prevent downstream migration of the device which could lead to leakage of blood into the aneurysmal sac. In this particular device, the illustrative barb 411 is designed to orient away from the heart in the direction of aortic blood flow; however, oppositely-oriented barbs may be used in certain other devices intended for aneurysm repair, such as a thoracic stent graft which would be placed in the aortic arch. The orientation of the barb in each of the disclosed examples is determined not only by where the device is placed in the body (i.e., accounting for the direction of blood or fluid flow), but by the type of barb as well, e.g., whether or not the barb includes a hooked end 429, as depicted in FIG. 100. In addition, barbs of different orientation may be used within the same device.

To form the helical coil 438 of the illustrative barb 411 of FIGs. 94-95, a length of 0.008-0.012" diameter wire (such as 0.401" spring stainless steel wire) is either machine wound or hand wound around the strut 415 such that the strut traverses the lumen 421 formed by the helical coil 438, thus forming a mechanical attachment 417 between the barb 411 and strut 415, best shown in FIG. 95. The helical windings 416 of the basal portion 413 have a first pitch 431 in which the windings 416 typically, but not necessarily, lie directly adjacent to one another.

Returning to FIG. 94, low-temperature silver solder, or some other bonding agent, is applied to the windings 416 of the basal portion 413 to form a permanent bond 418 that reinforces the mechanical attachment of the helical windings and secures the barb longitudinally along the strut 415. Besides the illustrative solder joint 418, alternative methods of forming a permanent bond 418 include welding or the use of adhesives. As depicted in FIGs. 94-95, helical coil 438 includes a winding 420 distal to those of the basal portion 413 and the point of union 419 between the barb 411 and strut. Referred to herein as the free winding 420 because it neither is soldered to the strut, or is generally in contact with the strut, except perhaps in an insignificant or incidental way, the free winding comprises the stress-dispersing region 414 of the barb. It should be noted that the free winding 420 does not necessarily completely encircle the substrate of origin or strut and may only constitute a partial winding. The free winding 420 is of a second pitch 432 that is typically greater (more loosely wound) than the first pitch 431 of windings 416 of the basal portion 413, although it is not essential that the basal winding 416 be closely adjacent to one another as depicted. By enlarging the radius of the winding 420, such that it is no longer contacting the strut 415, the bending stress is more evenly distributed than would be the case if there were a tighter winding (with less pitch), thereby increasing the fatigue life of the barb. Furthermore, the fact that the free winding 420 of the barb is not affixed to, nor is in contact with, the strut 415 allows the entire free winding 420 to freely flex and distribute most the bending forces over its entire length. This helps prevent the concentration of torsional and bending stresses at the point of union 419 where the barb 411 extends out from the solder joint 418, typically the most common location of barb fracture in the prior art barb illustrated in FIG. 93.

The anchoring portion 412 of the illustrative barb 411 of FIG. 94 comprises a straight section extending from the stress-dispersing portion such that the overall barb 411 length is about 5 mm, the typical range being 3-8 mm, depending on the stent used. The barb 411 extends at an angle 433 from the strut to facilitate the capture of anchoring tissue, the preferred post-deployment angle 433 being about 20-50°, e.g. 35°, in the illustrative example used to anchor the suprarenal stent of a AAA endovascular graft. Due to plastic deformation that may occur during loading of the device into a delivery system, such as a top cap, this angle may be initially formed at a somewhat larger angle 433 (i.e., 40-80°). The distal end 430 of the barb includes a bevel 435 to facilitate penetration of the vessel wall, with the sharp point being oriented toward the strut 415. The particular barb angle 433 and bevel 435 orientation are selected, in part, to ensure that the device 410 can be compressed to a smaller configuration and loaded into the top cap (not shown) of a delivery system and successful deployed therefrom such that the barb 411 does not deform or become caught within the cap, while still being able to resiliently extend outward to its expanded configuration and effectively engage tissue.

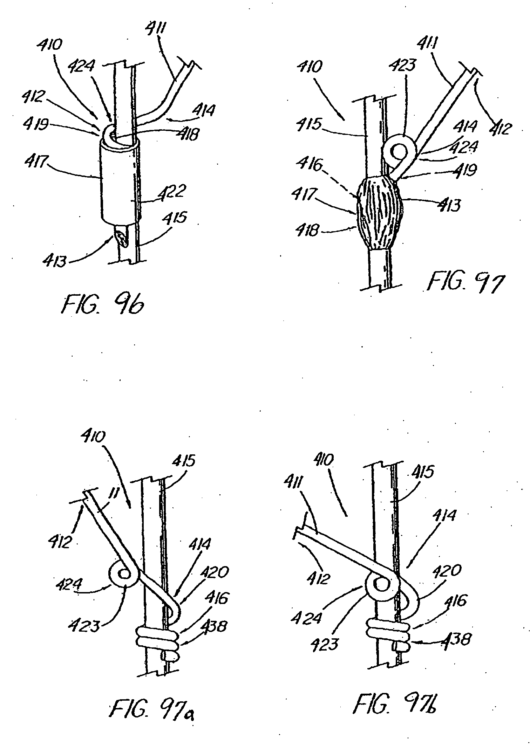

FIG. 96 depicts an alternative example of the present invention in which a short piece of metal cannula 422 is used as the mechanical attachment 417 to affix the barb 411 to the strut 415 of the intraluminal prosthesis 410. The basal section 413 of the barb 411 is secured against the strut 415 by the cannula 422, which is crimped over the barb and/or affixed using a solder joint 418 or some other means of fixation. At the point of union 419 of the barb 411 as it exits the region of attachment 417, the barb 411 assumes a series of bends or curves 424 that comprise the stress-dispersing region 414, after which the anchoring portion 412 extends outward at the appropriate angle from the strut 415. Alternatively, the cannula 422 can be used in combination with another type of mechanical attachment 417, such as the helical windings 416 of FIG. 95 in which the last winding 420 would comprise the stress-dispersing region 414.

FIGS. 97-98 depict alternative examples of stress-dispersing regions 414 of the barb 411 of the present invention which can be used with a variety of basal portion 413 configurations and types of attachments 417. In the embodiments of FIGs. 97, 97a, and 97b, stress-dispersing region 414 comprises a complete coiled loop 423 whereby the wire makes approximately a one and quarter turn between the basal portion 413 the anchoring portion 412 of the barb 411. The illustrative loop 423 provides a known mechanical advantage that it increases the range of flexibility at that bend, as evidenced by its use in certain medical devices, such as stents, and other devices with sharp bends (e.g., safety pins). Although the tighter-radius bends in general, can provide a site having an increased risk of fracture, this may be more than offset by the added flexibility of the barb, depending on the configuration. FIGs. 97a and 97b depict example that include both the free winding 420 as depicted in the examples of FIG. 94-95, as well as a coiled loop 423 that is located adjacent to the free winding 420. In the example of FIG. 97a, the coil is discrete from the free winding 420, wherein in the example of FIG. 97b, a portion of the coiled loop 423 originates from the free winding 420 such that they are essentially contiguous with one another. The combination of the coiled loop 423 and free winding 420 form a stress-dispersing region 414 having different flexibility characteristics that may be desirous in a particular application.

The example of FIG. 98 includes a generally U-shaped bend 424 that comprises the stress-dispersing region 414. The examples of FIGS. 97-98 are merely exemplary of the numerous configurations of bends 424 that can be utilized to redistribute bending stresses and reduce the risk of fracture. These and other undisclosed bends may used in combination within the stress-dispersing region 414 to further distribute the stress load of the implanted barb 411. Like the embodiments of FIGs. 97a-97b, the bends 424 may be combined with a free helical winding 420 for added flexibility.

FIG. 99 depicts an integrally formed barb 411 in which the barbed prosthesis 410 is partially or completely formed from a sheet of metal or other material, such as by laser cutting, eliminating the need for an separate attachment mechanism 417. The basal section 413 of the barb 411 basically comprises the point of union 419 between the strut portion 415 and the barb portion 411 from which it extends. In the illustrative embodiment of the present invention, the stress-dispersing region 414 comprises a series of bends 424, as well as a fillet 425 at the union 419 with the strut to further reduce stress concentration.

FIG. 100 depicts an example in which the prosthesis 410 includes a doubled-ended barb 411 having a first barb portion 436 that includes a first anchoring portion 412 and associated first stress-dispersing portion 414, and a second barb portion 437 that includes a second anchoring portion 426 and associated second stress-dispersing portion 427, all extending from a single basal portion 413, which in the illustrative example, comprises a helical coil 438 similar to that depicted in FIG. 95. Both the first free winding 420 extending from the first barb portion 436 and the second free winding 427 extending oppositely from the basal portion 413 are unattached to the strut 415 and free to flex and distribute any bending stresses therealong. Additionally, FIG. 100 also illustrates an alternative attachment means between the barb 411 and strut 415, wherein rather than a mechanical attachment 417 or bonding attachment 418, the helical coil 438 is allowed to slide along the strut 415, which may reduce the stress moment along the barb 411 in certain situations. To prevent the barb from sliding too far in either direction, a pair of stops 439, such as beads of solder, welded structure, burs formed in the strut 415, etc. are placed at either end of the basal portion 413. In the illustrative double barb 411, the first barb portion 436 includes a terminal hook 429 for anchoring the device to prevent migration due to blood or fluid flow, while the oppositely oriented second barb portion 437 includes a straight distal end 434. Alternatively, the exemplary double-ended barb 411 can be modified to include other disclosed configurations of the basal, stress-dispersing or anchoring portions or regions 412,413,414 of the barb 411 or any appropriate means of attachment to the strut 415.

Any other undisclosed or incidental details of the construction or composition of the various elements of the disclosed embodiment of the present invention are not believed to be critical to the achievement of the advantages of the present invention, so long as the elements possess the attributes needed for them to perform as disclosed. The selection of these and other details of construction are believed to be well within the ability of one of even rudimentary skills in this area, in view of the present disclosure. Illustrative embodiments of the present invention have been described in considerable detail for the purpose of disclosing a practical, operative structure whereby the invention may be practiced advantageously. The designs described herein are intended to be exemplary only. The novel characteristics of the invention may be incorporated in other structural forms without departing from the scope of the invention. The invention encompasses embodiments both comprising and consisting of the elements described with reference to the illustrative embodiments. Unless otherwise indicated, all ordinary words and terms used herein shall take their customary meaning as defined in The New Shorter Oxford English Dictionary, 1993 edition. All technical terms shall take on their customary meaning as established by the appropriate technical discipline utilized by those normally skilled in that particular art area. All medical terms shall take their meaning as defined by Stedman's Medical Dictionary, 27th edition.

## Claims

1. A method for manufacturing a barbed stent, comprising:
providing a sheet of stent material (310);
cutting the sheet to form a stent segment (322) with integral barbs (314) extending therefrom;
forming the stent segment (322) into a final stent shape (355);
the method **characterized in that**, in a step performed prior to the step in which the stent segment (322) is formed into a final stent shape (355), the stent segment (322) is oriented such that the barbs (314) point in a predetermined direction relative to a longitudinal axis (359) of the final stent shape (355) in order to engage an adjacent vessel wall obviating the need for bending the barbs (314) relative to the stent segment (322) during the forming.

2. The method of claim 1, **characterized by** connecting a first end (330) of the stent segment (322) to a second end (332) of the stent segment (322).

3. The method of claim 1, **characterized by** soldering a first end (330) of the stent segment (322) to a second end (332) of the stent segment (322).

4. The method of claim 1, **characterized by** polishing the stent segment (322).

5. The method of claim 1 or 2, **characterized by** electropolishing the stent segment (322).

6. The method of claim 1, **characterized in that** the cutting is performed with a laser.

7. The method of claim 1, 2 or 6, **characterized in that** forming the stent segment (322) into a final stent shape (355) comprises bending the stent segment (322) into a zigzag shape.

8. The method of claim 1,2 or 6, **characterized in that** forming the stent segment (322) into a final stent shape (355) comprises bending the stent segment (322) into a zigzag shape having alternating struts (328) and bends (326), so that at least, one of the barbs (314) is integral with at least every other strut (328).

9. The method of claim 1, 2 or 6, **characterized in that** forming the stent segment (322) into a final stent shape (355) comprises bending the stent segment (322) into a sinusoidal shape.

10. The method of claim 1, 2 or 6, **characterized in that** the stent material (310) is stainless steel.

11. A barbed stent (355) for deployment within the body of a patient, comprising:
a stent segment (322) having at least one bend (326), wherein each bend (326) connects to at least two struts (328); and
barbs (314) pointing in a predetermined direction relative to a longitudinal axis (359) of the stent;
wherein the barbs (314) are integral with the stent segment (322); **characterised in that** each barb is unbent with respect to the stent segment and is free of weakening due to bending.

12. The stent (355) of claim 11, **characterized in that** the stent is in a zigzag shape.

13. The stent (355) of claim 12, **characterized in that** each of the barbs (314) forms an acute angle to the longitudinal axis (359).

14. The sent (355) of claim 11 or 12, **characterized in that** the barbs (314) are positioned on the at least one bend (326).

15. The stent (355) of claim 11 or 12, **characterized in that** the barbs (314) are positioned on the at least two struts (328).

16. The stent (355) of claim 11 or 12, **characterized in that** the stent segment (322) comprises stainless steel.

17. The stent (360) of claim 11 or 12, **characterized in that** the stent is adjacent to a proximal end of an endoluminal prosthesis (370).

18. The stent (372) of claim 17, **characterized in that** the at least two struts (328) extend away from the proximal end of the endoluminal prosthesis (370) in a proximal direction.

19. The stent (372) of claim 18, **characterized in that** the endoluminal prosthesis (370) is adapted to be deployed at least partially within the aorta, so that the stent (372) can extend at least partially above a renal artery when the prosthesis (370) is implanted.

20. The stent (360) of claim 19, **characterized in that** the prosthesis (362) is a bifurcated aortic prosthesis,

## Patentansprüche

1. Verfahren zum Herstellen eines Stents mit Spitzen, umfassend:
Bereitstellen eines Bogens von Stentmaterial (310);
Schneiden des Bogens, um ein Stentsegment (322) zu bilden, von dem integrale Spitzen (314) vorragen;
Formen des Stentsegments (322) zu einer fertigen Stentform (355);
wobei das Verfahren **dadurch gekennzeichnet ist, dass** bei einem Schritt, der vor dem Schritt durchgeführt wird, bei dem das Stentsegment (322) zu einer fertigen Stentform (355) geformt wird, das Stentsegment (322) so ausgerichtet wird, dass die Spitzen (314) in eine vorbestimmte Richtung bezogen auf die Längsachse (359) der fertigen Stentform (355) zeigen, um in eine angrenzende Gefäßwand einzugreifen, wodurch die Notwenigkeit umgangen wird, die Spitzen (314) während der Herstellung relativ zu dem Stentsegment (322) zu biegen.

2. Verfahren gemäß Anspruch 1, **gekennzeichnet durch** Verbinden eines ersten Endes (330) des Stentsegments (322) mit einem zweiten Ende (332) des Stentsegments (322).

3. Verfahren gemäß Anspruch 1, **gekennzeichnet durch** Löten eines ersten Endes (330) des Stentsegments (322) an ein zweites Ende (332) des Stentsegments (322).

4. Verfahren gemäß Anspruch 1, **gekennzeichnet durch** Polieren des Stentsegments (322).

5. Verfahren gemäß Anspruch 1 oder 2, **gekennzeichnet durch** Elektropolieren des Stentsegments (322).

6. Verfahren gemäß Anspruch 1, **dadurch gekennzeichnet, dass** das Schneiden mit einem Laser durchgeführt wird.

7. Verfahren gemäß Anspruch 1, 2 oder 6, **dadurch gekennzeichnet, dass** das Formen des Stentsegments (322) zu einer fertigen Stentform (355) das Biegen des Stentsegments (322) zu einer Zickzack-Form umfasst.

8. Verfahren gemäß Anspruch 1, 2 oder 6, **dadurch gekennzeichnet, dass** das Formen des Stentsegments (322) zu einer fertigen Stentform (355) das Biegen des Stentsegments (322) zu einer Zickzack-Form mit abwechselnden Streben (328) und Biegungen (326) umfasst, so dass mit wenigstens jeder zweiten Strebe (328) wenigstens eine der Spitzen (314) integral ist.

9. Verfahren gemäß Anspruch 1, 2 oder 6, **dadurch gekennzeichnet, dass** das Formen des Stentsegments (322) zu einer fertigen Stentform (355) das Biegen des Stentsegments (322) in eine sinusartige Form umfasst.

10. Verfahren gemäß Anspruch 1, 2 oder 6, **dadurch gekennzeichnet, dass** das Stentmaterial (310) rostfreier Stahl ist.

11. Stent mit Spitzen (355) zum Einsetzen in den Körper eines Patienten, umfassend:
ein Stentsegment mit wenigstens einer Biegung (326), wobei jede Biegung (326) wenigstens zwei Streben (328) verbindet; und
Spitzen (314), die in eine vorbestimmte Richtung bezogen auf die Längsachse (359) des Stents zeigen;
wobei die Spitzen (314) integral mit dem Stentsegment (322) sind;
**dadurch gekennzeichnet, dass** jede Spitze bezogen auf nach Stentsegment nichtgebogen ist und von Schwächung durch Biegen frei ist.

12. Stent (355) gemäß Anspruch 11, **dadurch gekennzeichnet, dass** der Stent eine Zickzack-Form aufweist.

13. Stent (355) gemäß Anspruch 12, **dadurch gekennzeichnet, dass** jede der Spitzen (314) einen spitzen Winkel zu der Längsachse (359) bildet.

14. Stent (355) gemäß Anspruch 11 oder 12, **dadurch gekennzeichnet, dass** die Spitzen (314) an der wenigstens einen Biegung (326) angeordnet sind.

15. Stent (355) gemäß Anspruch 11 oder 12, **dadurch gekennzeichnet, dass** die Spitzen (314) an den wenigstens zwei Streben(328) angeordnet sind.

16. Stent (355) gemäß Anspruch 11 oder 12, **dadurch gekennzeichnet, dass** das Stentsegment (322) rostfreien Stahl umfasst.

17. Stent (360) gemäß Anspruch 11 oder 12, **dadurch gekennzeichnet, dass** der Stent benachbart zu einem proximalen Ende einer endoluminalen Prothese (370) angeordnet ist.

18. Stent (372) gemäß Anspruch 17, **dadurch gekennzeichnet, dass** die wenigstens zwei Streben (328) von dem proximalen Ende der endoluminalen Prothese (370) in einer proximalen Richtung wegragen.

19. Stent (372) gemäß Anspruch 18, **dadurch gekennzeichnet, dass** die endoluminale Prothese (370) dafür ausgelegt ist, wenigstens teilweise in die Aorta eingesetzt zu werden, so dass der Stent (372) wenigstens teilweise über eine Nierenarterie vorragen kann, wenn die Prothese (370) implantiert wird.

20. Stent (360) gemäß Anspruch 19, **dadurch gekennzeichnet, dass** die Prothese (370) eine gegabelte Aortaprothese ist.

## Revendications

1. Procédé de fabrication d'un stent à barbes, comprenant les étapes suivantes:
fournir une feuille d'un matériau de stent (310);
couper la feuille de manière à former un segment de stent (322) avec des barbes monoblocs (314) qui s'étendent à partir de celui-ci;
former le segment de stent (322) à une forme de stent finale (355);
le procédé étant **caractérisé en ce que**, dans une étape exécutée avant l'étape au cours de laquelle le segment de stent (322) est formé à une forme de stent finale (355), le segment de stent (322) est orienté de telle sorte que les barbes (314) pointent dans une direction prédéterminée par rapport à un axe longitudinal (359) de la forme de stent finale (355) dans le but d'engager une paroi de vaisseau adjacente sans qu'il soit nécessaire de plier les barbes (314) par rapport au segment de stent (322) pendant le formage.

2. Procédé selon la revendication 1, **caractérisé par** la connexion d'une première extrémité (330) du segment de stent (322) à une deuxième extrémité (332) du segment de stent (322).

3. Procédé selon la revendication 1, **caractérisé par** le brasage d'une première extrémité (330) du segment de stent (322) à une deuxième extrémité (332) du segment de stent (322).

4. Procédé selon la revendication 1, **caractérisé par** le polissage du segment de stent (322).

5. Procédé selon la revendication 1 ou 2, **caractérisé par** l'électro-polissage du segment de stent (322).

6. Procédé selon la revendication 1, **caractérisé en ce que** la coupe est exécutée à l'aide d'un laser.

7. Procédé selon la revendication 1, 2 ou 6, **caractérisé en ce que** le formage du segment de stent (322) à une forme de stent finale (355) comprend le pliage du segment de stent (322) en une forme de zigzag.

8. Procédé selon la revendication 1, 2 ou 6, **caractérisé en ce que** le formage du segment de stent (322) à une forme de stent finale (355) comprend le pliage du segment de stent (322) en une forme de zigzag présentant des entretoises (328) et des courbes (326) alternées, de telle sorte qu'au moins une des barbes (314) soit monobloc avec au moins une entretoise (328) sur deux.

9. Procédé selon la revendication 1, 2 ou 6, **caractérisé en ce que** le formage du segment de stent (322) à une forme de stent finale (355) comprend le pliage du segment de stent (322) en une forme sinusoïdale.

10. Procédé selon la revendication 1, 2 ou 6, **caractérisé en ce que** le matériau de stent (310) est l'acier inoxydable.

11. Stent à barbes (355) à déployer à l'intérieur du corps d'un patient, comprenant:
un segment de stent (322) présentant au moins une courbe (326), dans lequel chaque courbe (326) se connecte à au moins deux entretoises (328); et
des barbes (314) qui pointent dans une direction prédéterminée par rapport à un axe longitudinal (359) du stent;
dans lequel les barbes (314) sont monoblocs avec le segment de stent (322),
**caractérisé en ce que** chaque barbe est non pliée par rapport au segment de stent et n'est pas affaiblie par le pliage.

12. Stent (355) selon la revendication 11, **caractérisé en ce que** le stent est en forme de zigzag.

13. Stent (355) selon la revendication 12, **caractérisé en ce que** chacune des barbes (314) forme un angle aigu par rapport à l'axe longitudinal (359).

14. Stent (355) selon la revendication 11 ou 12, **caractérisé en ce que** les barbes (314) sont positionnées sur ladite au moins une courbe (326).

15. Stent (355) selon la revendication 11 ou 12, **caractérisé en ce que** les barbes (314) sont positionnées sur lesdites au moins deux entretoises (328).

16. Stent (355) selon la revendication 11 ou 12, **caractérisé en ce que** le segment de stent (322) est constitué d'acier inoxydable.

17. Stent (360) selon la revendication 11 ou 12, **caractérisé en ce que** le stent est adjacent à une extrémité proximale d'une prothèse endoluminale (370).

18. Stent (372) selon la revendication 17, **caractérisé en ce que** lesdites au moins deux entretoises (328) s'étendent à l'écart de l'extrémité proximale de la prothèse endoluminale (370) dans une direction proximale.

19. Stent (372) selon la revendication 18, **caractérisé en ce que** la prothèse endoluminale (370) est adaptée pour être déployée au moins partiellement à l'intérieur de l'aorte, de telle sorte que le stent (372) puisse s'étendre au moins partiellement au-dessus d'une artère rénale lorsque la prothèse (370) est implantée.

20. Stent (360) selon la revendication 19, **caractérisé en ce que** la prothèse (362) est une prothèse aortique bifurquée.
